# EUROPEAN PATENT SPECIFICATION

(11) **EP 3 698 710 B1**
(45) Date of publication and mention of the grant of the patent: **09.07.2025**
(21) Application number: 18867592.0
(22) Date of filing: 19.10.2018
(51) Int. Cl.: A61B 5/291, A61B 5/374, A61H 1/02

(54) **BRAIN WAVE MEASUREMENT SYSTEM, BRAIN WAVE MEASUREMENT METHOD, PROGRAM, AND NON-TRANSITORY RECORDING MEDIUM**
HIRNSTROMMESSSYSTEM, HIRNSTROMMESSVERFAHREN, PROGRAMM UND NICHT-TRANSITORISCHES AUFZEICHNUNGSMEDIUM
APPAREIL D'ANALYSE D'ONDES CÉRÉBRALES, PROCÉDÉ D'ANALYSE D'ONDES CÉRÉBRALES, PROGRAMME ET SUPPORT D'ENREGISTREMENT NON TRANSITOIRE

(30) Priority: 20.10.2017 JP 2017204093
(43) Date of publication of application: 26.08.2020
(73) Proprietor: LIFESCAPES Inc., Tokyo (JP); Keio University, Tokyo 108-8345 (JP)
(72) Inventor: USHIBA, Junichi, Yokohama, Kanagawa 223-8522 (JP); OKUYA, Teruhisa, Osaka-shi, Osaka 5406207 (JP); MORIKAWA, Koji, Osaka-shi, Osaka 540-6207 (JP); IWAKAWA, Mikio, Osaka-shi, Osaka 540-6207 (JP); HIRAMOTO, Miho, Tokyo 160-8582 (JP); OKUYAMA, Kohei, Tokyo 160-8582 (JP)
(74) Representative: Appelt, Christian W.
(86) International application number: PCT/JP2018/038932
(87) International publication number: WO 2019/078328

(56) References cited:
- WO-A1-2014/149709
- WO-A1-2017/060804
- JP-A- 2002 153 435
- JP-A- 2004 350 797
- JP-A- 2007 105 383
- JP-A- 2016 513 534
- US-A1- 2011 130 675
- US-A1- 2014 058 528
- US-A1- 2016 000 382

## Description

### Technical Field

The present disclosure generally relates to an electroencephalogram (EEG) measurement system, an electroencephalogram measurement method, a program, and a non-transitory storage medium. More particularly, the present disclosure relates to an electroencephalogram measurement system, electroencephalogram measurement method, program, and non-transitory storage medium, all of which are configured or designed to acquire electroencephalogram information representing a subject-specific electroencephalogram obtained by an electrode unit that is placed on a region of interest (ROI) forming part of the subject's head.

### Background Art

Various techniques for using subject-specific electroencephalogram information have been proposed.

For example, Patent Literature 1 discloses a technique for inferring, based on an electroencephalogram measured when a subject (testee) is listening to a musical tune, whether or not he or she has ever listened to that musical tune. According to the technique disclosed in Patent Literature 1, the electroencephalogram specific to a testee who is listening to a musical tune and who may or may not have ever listened to that tune is measured to acquire electroencephalogram information (testee electroencephalogram data), and a primary feature derived from the electroencephalogram information is collated with a modeled electroencephalogram pattern, thereby inferring, based on the result of the collation, whether or not the testee has ever listened to the musical tune.

According to the method disclosed in Patent Literature 1, the subject-specific electroencephalogram is measured and recorded with the subject 5 made to wear an electrode cap with twelve electrodes, keep his or her eyes closed, and minimize his or her body movement while his or her electroencephalogram is being recorded in order to reduce artifacts or noise. In addition, Patent Literature 1 also teaches subjecting an electroencephalogram signal to processing for reducing artifacts to be caused by the subject's unconscious body movement and eye movement.

According to the method disclosed in Patent Literature 1, however, artifacts may cause a significant decline in the accuracy of the electroencephalogram measured. This is because it is impossible to reduce artifacts sufficiently just by making the subject close his or her eyes and minimize his or her body movement and also difficult to remove artifacts completely through signal processing.

US 2011/130675 A1 discloses a quantitative electroencephalogram (QEEG) monitor and system capable of monitoring and displaying simultaneously neuropathological characteristic and activity of both sides of a subject's brain.

US 2014/058528 A1 discloses a noninvasive brain computer interface (BCI) system including an electroencephalography (EEG) electrode array configured to acquire EEG signals generated by a subject. The subject observes movement of a stimulus.

US 2016/000382 A1 discloses a method for monitoring EEG signals of patients during anaesthesia , involving updating extracted parameters in a display screen and an algorithm used to determine an event indicator in response to user selection.

### Citation List

### Patent Literature

Patent Literature 1: JP 2017-42562 A

### Summary of Invention

In view of the foregoing background, it is therefore an object of the present disclosure to provide an electroencephalogram measurement system, electroencephalogram measurement method, program, and non-transitory storage medium, all of which are configured or designed to reduce the chances of artifacts causing a decline in the accuracy of the electroencephalogram measured.

An electroencephalogram measurement system, electroencephalogram measurement method, program, and non-transitory storage medium according to the present invention are defined in independent claims 1, 4, 5 and 6, respectively. Preferred embodiments are defined in claims 2 and 3.

### Brief Description of Drawings

FIG. 1 schematically illustrates how an electroencephalogram measurement system according to a first embodiment and a rehabilitation support system including the electroencephalogram measurement system may be used;
FIG. 2 is a schematic front view illustrating how the headset of the electroencephalogram measurement system may be used;
FIG. 3 is a block diagram illustrating a configuration for the electroencephalogram measurement system and the rehabilitation support system;
FIG. 4 illustrates an exemplary training screen of the electroencephalogram measurement system;
FIG. 5 illustrates an exemplary setting screen of the electroencephalogram measurement system;
FIG. 6 is a graph showing how to determine a threshold value with respect to an activation level of the electroencephalogram measurement system;
FIG. 7 illustrates an exemplary calibration screen of the electroencephalogram measurement system;
FIG. 8 illustrates an exemplary selection screen of the electroencephalogram measurement system;
FIG. 9 illustrates an exemplary calibration result screen of the electroencephalogram measurement system;
FIG. 10 is a schematic representation illustrating how the electroencephalogram measurement system performs artifact decision processing;
FIG. 11 is a flowchart showing the procedure in which the electroencephalogram measurement system performs the artifact decision processing;
FIG. 12 illustrates an exemplary calibration result screen of an electroencephalogram measurement system according to a second embodiment;
FIG. 13 is a waveform diagram showing an electroencephalogram derived by bipolar derivation and an electroencephalogram derived by monopolar derivation in the electroencephalogram measurement system; and
FIG. 14 conceptually illustrates the flow of artifact decision processing performed by the electroencephalogram measurement system.

### Description of Embodiments

### (First embodiment)

### (1) Overview

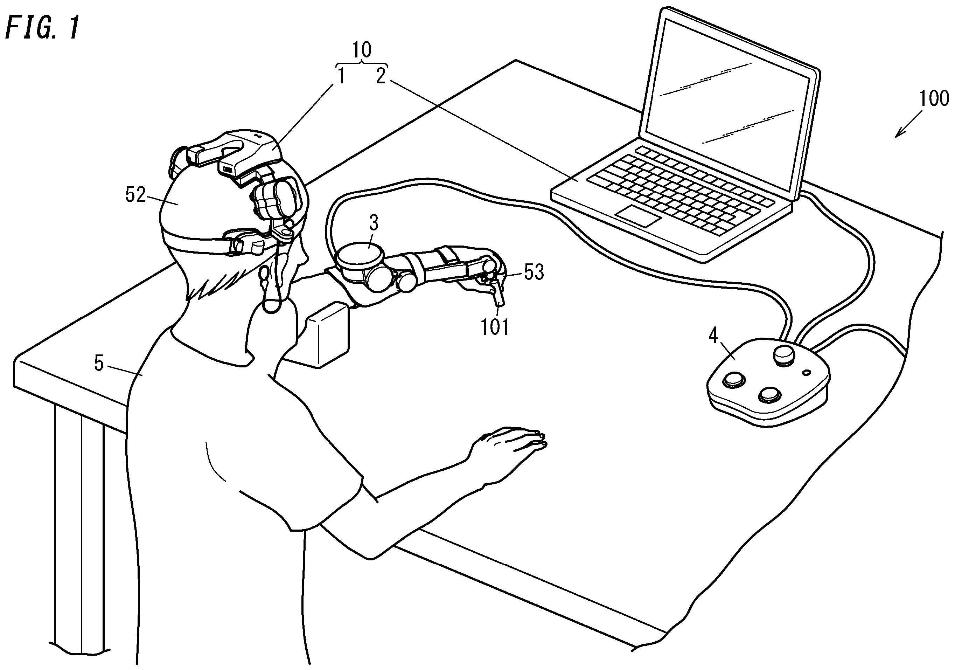

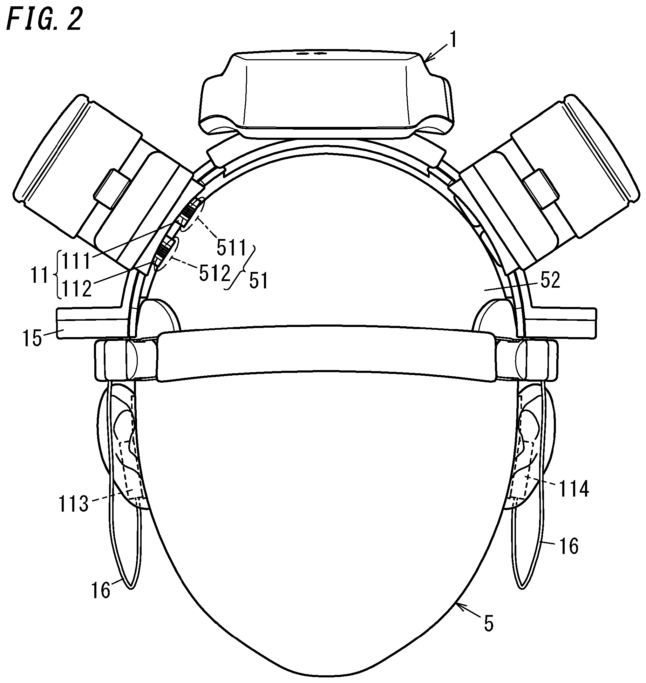

An overview of an electroencephalogram measurement system 10 according to an exemplary embodiment will be described with reference to FIGS. 1 and 2.

The electroencephalogram measurement system 10 according to this embodiment is a system for measuring an electroencephalogram specific to a subject 5, and acquires electroencephalogram information representing a subject-specific electroencephalogram obtained by an electrode unit 11 placed on a region of interest 51 that forms part of the subject's 5 head 52. As used herein, the "electroencephalogram (EEG)" refers to a waveform recorded by deriving, out of a human's body, electrical signals (action potentials) generated by (groups of) nerve cells (or neurons) of a human brain. Also, as used herein, an "electroencephalogram" refers to, unless otherwise stated, an on-scalp electroencephalogram obtained by recording, using the electrode unit 11 worn by the subject 5 on his or her body surface, a comprehensive action potential of a great many groups of neurons (that form a neural network) of the cerebral cortex.

The electroencephalogram measurement system 10 includes a headset 1 with the electrode unit 11 and an information processor 2. The headset 1 is worn by a subject 5 on his or her head 52 with the electrode unit 11 brought into contact with his or her head 52 surface (i.e., scalp). According to the present disclosure, the electrode unit 11 is mounted on paste (electrode paste) applied onto the surface of the head 52, and thereby comes into contact with the surface of the head 52. In this case, the electrode unit 11 comes into direct contact with (i.e., not via the subject's hairs) the surface of the head 52 by pushing the hairs aside. Naturally, the electrode unit 11 may come into direct contact with the surface of the head 52 with no paste applied between them. That is to say, according to the present disclosure, "to bring the electrode unit 11 into contact with the surface of the head 52" refers to not only bringing the electrode unit 11 into direct contact with the surface of the head 52 but also bringing the electrode unit 11 into indirect contact with the surface of the head 52 with some intermediate interposed between the electrode unit 11 and the surface of the head 52. The intermediate does not have to be paste but may also be a gel with electrical conductivity.

The headset 1 measures the electroencephalogram specific to the subject 5 by having the electrode unit 11 measure the action potential of the subject's 5 brain, thereby generating electroencephalogram information representing the electroencephalogram. The headset 1 may transmit the electroencephalogram information to the information processor 2 by wireless communication, for example. In response, the information processor 2 subjects the electroencephalogram information acquired from the headset 1 to various types of processing, or displays the electroencephalogram information thereon.

In the following description of embodiments, a situation where the electroencephalogram measurement system 10 is used in a rehabilitation support system 100 for supporting the subject 5 in his or her rehabilitation will be described. That is to say, the rehabilitation support system 100 includes the electroencephalogram measurement system 10 according to this embodiment. The rehabilitation support system 100 further includes an exercise assisting device 3 and a controller 4. The exercise assisting device 3 assists the subject 5 with his or her exercise by applying at least one of a mechanical stimulus or an electrical stimulus to the subject 5. The controller 4 controls the exercise assisting device 3 based on the electroencephalogram information acquired by the electroencephalogram measurement system 10.

This rehabilitation support system 100 supports a subject 5, who suffers from either a motor paralysis or a decline in motor function in some region of his or her body due to some brain disease such as cerebral apoplexy (stroke) or a traffic accident, in his or her rehabilitation by exercise therapy. Such a subject 5 may either be unable to do, or show a decline in the physical ability to do well, a voluntary movement, which is a movement that the subject 5 does of his or her own will or by intention. As used herein, the "exercise therapy" refers to a method for recovering a voluntary movement function for an affected region of the subject's 5 body by making the subject 5 exercise his or her region that is either unable to do, or shows a decline in the physical ability do well, such a voluntary movement (hereinafter referred to as "affected region").

In the following description of embodiments, the rehabilitation support system 100 is supposed to be used, for example, to support the subject 5 in his or her rehabilitation to recover the function of his or her left hand fingers 53. That is to say, in this case, the subject's 5 left hand fingers are his or her affected region. However, this is only an example and should not be construed as limiting. Alternatively, the rehabilitation support system 100 may also be used to support the subject 5 in his or her rehabilitation to recover the function of his or her right hand fingers.

The rehabilitation support system 100 supports the subject 5 in his or her voluntary movement by having the exercise assisting device 3, which the subject 5 wears on his or her left hand, apply at least one of a mechanical stimulus or an electrical stimulus to his or her left hand when the subject 5 does the voluntary movement using his or her left hand fingers 53. This allows, just like a situation where a medical staff such as a physical therapist or an occupational therapist supports the subject 5 in his or her voluntary movement by holding the subject's 5 hand fingers 53, the rehabilitation support system 100 to support him or her in the voluntary movement. Thus, the rehabilitation support system 100 is able to provide rehabilitation by exercise therapy more effectively than in a situation where the subject 5 does the voluntary movement by him- or herself, to say nothing of a situation where some medical staff provides the support.

Meanwhile, to provide support for such rehabilitation, the rehabilitation support system 100 suitably assists, using the exercise assisting device 3, the subject 5 with his or her voluntary movement when the subject 5 is going to do the voluntary movement on his or her own. The rehabilitation support system 100 assists, when the subject 5 is going to do voluntary movement, him or her in the voluntary movement using the exercise assisting device 3 by operating the exercise assisting device 3 in coordination with the subject's 5 electroencephalogram (electroencephalogram information) measured by the electroencephalogram measurement system 10. In other words, the rehabilitation support system 100 provides rehabilitation by exercise therapy by using the brain-machine interface (BMI) technology for operating a machine (such as the exercise assisting device 3) based on the brain activity (electroencephalogram).

When the subject 5 is going to do voluntary movement (i.e., while the subject 5 is doing the voluntary movement), a characteristic variation may arise in his or her electroencephalogram. That is to say, when the subject 5 plans to do (or imagines doing) the voluntary movement, a brain region corresponding to the region that should be exercised to do the voluntary movement may be activated. Examples of such brain regions include a somatosensory motor cortex. Supporting the subject 5 in his or her voluntary movement using the exercise assisting device 3 at the timing when the brain region is activated would make the rehabilitation even more effective. Such brain region activation may be detected as a characteristic variation in electroencephalogram. Thus, the rehabilitation support system 100 starts supporting the subject 5 in his or her voluntary movement using the exercise assisting device 3 at the timing when this characteristic variation arises in the electroencephalogram specific to the subject 5. Note that such a characteristic variation may arise in the electroencephalogram even if the voluntary movement is not actually carried out but when the subject 5 imagines doing the voluntary movement (i.e., plans to do the movement). That is to say, this characteristic variation may arise in the electroencephalogram even if the voluntary movement is not actually carried out but when the subject 5 plans to do, or imagines doing, the voluntary movement to activate the corresponding brain region. Therefore, the rehabilitation support system 100 may also support even a subject 5, who is unable to do the voluntary movement, in his or her attempt to do the voluntary movement.

The rehabilitation support system 100 with such a configuration is able to provide effective rehabilitation by exercise therapy for the subject 5 while lightening the workload on medical staff. In addition, this rehabilitation support system 100 eliminates the variation in timing to start supporting the subject 5 in his or her voluntary movement due to a human factor such as the skill level of the medical staff who needs to support the subject 5 in his or her voluntary movement, thus reducing the variation in the effect of rehabilitation. In particular, the rehabilitation support system 100 is able to start supporting the subject 5 in his or her voluntary movement at the timing when a characteristic variation arises in his or her specific electroencephalogram (i.e., the timing when the brain region is actually activated). As can be seen, this rehabilitation support system 100 allows the subject 5 to start training at the timing when his or her brain activity starts, thus contributing to learning and establishing right brain activity. Among other things, it is difficult for even the subject 5 him- or herself and the medical staff to determine whether or not such a characteristic variation has arisen in his or her electroencephalogram. Thus, using this rehabilitation support system 100 provides highly effective rehabilitation that is usually difficult to realize by either the subject 5 or the medical staff alone.

In the embodiment to be described below, when the subject 5 uses the rehabilitation support system 100, the subject 5 is supposed to be accompanied by some medical staff such as a physical therapist or an occupational therapist and the rehabilitation support system 100 is supposed to be operated by the medical staff. However, the subject 5 who uses the rehabilitation support system 100 does not have to be accompanied by medical staff. Alternatively, the rehabilitation support system 100 may be operated by either the subject 5 him- or herself or his or her family member as well.

The electroencephalogram measurement system 10 according to this embodiment is used in the rehabilitation support system 100 in order to detect an electroencephalogram with a characteristic variation that arises when the subject 5 is going to do the voluntary movement (i.e., when the subject 5 plans to do the voluntary movement). As will be described in detail later in the "(2) Rehabilitation support system" section, the electroencephalogram measurement system 10 detects, as the characteristic variation, a variation caused due to event-related desynchronization (ERD) in the intensity of the electroencephalogram within a particular frequency band. As used herein, the "event-related desynchronization" refers to a decline in power falling within a particular frequency band of the electroencephalogram representing a brain wave measured in the vicinity of a motor area during the voluntary movement (or when the subject 5 just imagines doing the voluntary movement). As used herein, the phrase "during the voluntary movement" refers to a process that begins when the subject 5 plans to do (or imagines doing) the voluntary movement and ends when the voluntary movement is either done successfully or ends up in failure. The "event-related desynchronization" may be triggered, during the voluntary movement, by the subject's plan to do the voluntary movement (or his or her image of doing the voluntary movement). The frequency bands in which the event-related desynchronization causes a decline in power are mainly an α wave range (such as a frequency band from 8 Hz to less than 13 Hz) and a β wave range (such as a frequency band from 13 Hz to less than 30 Hz).

Nevertheless, the frequency band in which the event-related desynchronization causes a decline in power and the magnitude of the decline in power are not constant but vary according to the subject's 5 attributes (such as age and sex), affected region, affected condition, and other individual differences. Therefore, the electroencephalogram as the target of detection for the electroencephalogram measurement system 10 (i.e., the electroencephalogram with a characteristic variation that may arise when the subject 5 plans to do the voluntary movement) is not determined uniformly but may vary from one subject 5 to another. Thus, the electroencephalogram measurement system 10 is configured to perform the calibration processing for analyzing the electroencephalogram information, i.e., determining various types of parameters for use to detect the electroencephalogram as the target of detection.

In this case, if the electroencephalogram information includes any artifacts, the electroencephalogram measurement accuracy may decrease due to the artifacts. As used herein, the "artifacts" refer to various types of non-electroencephalogram phenomena, which may form unintentional parts of the electroencephalogram information acquired by the electroencephalogram measurement system 10, and may constitute noise components with respect to the electroencephalogram. Examples of such artifacts that may form unintentional parts of the electroencephalogram information include components deriving from eye movement such as a wink, components deriving from the body movements such as teeth grinding and neck turns, and components deriving from facial muscle movements. If the calibration processing is carried out using the electroencephalogram information with these types of artifacts, then the calibration processing would be performed with the electroencephalogram measurement accuracy decreased due to the artifacts, thus causing a decline in the accuracy of the parameters. Thus, to determine the parameters accurately, the electroencephalogram measurement system 10 suitably performs the calibration processing based on the electroencephalogram information free of artifacts.

The electroencephalogram measurement system 10 according to this embodiment has the capability of determining, based on the electroencephalogram information acquired from the electrode unit 11, whether or not there are any artifacts and outputting the decision. The decision may be output as a text message displayed, a verbal message emitted, a page printed out, a file written into a non-transitory storage medium, or data transmitted to a telecommunications device. This allows the user to determine, based on the decision output, whether or not there are any artifacts, thus reducing the chances of the artifacts causing a decline in the electroencephalogram measurement accuracy by removing electroencephalogram information with the artifacts. Consequently, outputting the decision indicating whether or not there are any artifacts enables the electroencephalogram measurement system 10 to carry out the calibration processing based on only the electroencephalogram information free of artifacts.

### (2) Rehabilitation support system

Next, a rehabilitation support system 100 according to this embodiment will be described in further detail.

As shown in FIG. 1, the rehabilitation support system 100 includes the electroencephalogram measurement system 10, the exercise assisting device 3, and the controller 4.

As described above, the electroencephalogram measurement system 10 according to this embodiment includes the headset 1 and the information processor 2.

As shown in FIG. 2, the headset 1 is worn by the subject 5 on his or her head 52. The headset 1 includes the electrode unit 11. The electrode unit 11 is placed on a region of interest 51, which forms part of the subject's 5 head 52. Specifically, the headset 1 has the subject's 5 electroencephalogram measured by the electrode unit 11 that is brought into contact with the region of interest 51 defined on an area of the surface (scalp) of the subject's 5 head 52, thereby generating electroencephalogram information representing the electroencephalogram.

The information processor 2 includes, as its main constituent element, a computer system such as a personal computer. The information processor 2 receives the electroencephalogram information from the headset 1 via wireless communication, and performs various types of processing on the electroencephalogram information. In this embodiment, detection of an electroencephalogram with a characteristic variation that may arise when the subject 5 plans to do (or imagines doing) the voluntary movement, the calibration processing, and other types of processing are performed by the information processor 2.

When the subject 5 plans to do (or imagines doing) the voluntary movement, the electroencephalogram measured usually comes to have a characteristic variation that represents a brain wave produced in the motor area corresponding to a body region where the voluntary movement is conducted. Thus, the electroencephalogram measurement system 10 regards, as the target of measurement, the electroencephalogram detected from around the motor area corresponding to the affected region as the target of rehabilitation. In this case, the motor area corresponding to left hand fingers is located on the right side of the brain and the motor area corresponding to right hand fingers is located on the left side of the brain. That is why when the subject's 5 left hand fingers 53 are the target of rehabilitation as in this embodiment, the electroencephalogram obtained by the electrode unit 11 that is brought into contact with the right side of the subject's 5 head 52 is the target of measurement for this electroencephalogram measurement system 10. That is to say, the electrode unit 11 is placed on a region of interest 51 that forms part of the right surface of the subject's 5 head 52 as shown in FIG. 2. For example, the electrode unit 11 is placed at a location designated by the mark "C4" according to the international 10-20 system. On the other hand, when the subject's 5 right hand fingers are the target of rehabilitation, the electrode unit 11 is placed on a region of interest that forms part of the left surface of the subject's 5 head 52. For example, the electrode unit 11 may be placed at a location designated by the mark "C3" according to the international 10-20 system in that case.

On detecting an electroencephalogram with a characteristic variation that may arise when the subject 5 plans to do voluntary movement, the electroencephalogram measurement system 10 outputs a control signal for controlling the exercise assisting device 3. That is to say, in this rehabilitation support system 100, generation of a control signal for controlling the exercise assisting device 3 is triggered by detection by the electroencephalogram measurement system 10 of an electroencephalogram with a characteristic variation that may arise when the subject 5 plans to do voluntary movement. Thus, this rehabilitation support system 100 allows the exercise assisting device 3 to assist the subject 5 with his or her voluntary movement when the subject 5 is going to do the voluntary movement. The electroencephalogram measurement system 10 will be described in further detail later in the "(3) Electroencephalogram measurement system" section.

The exercise assisting device 3 is a device for assisting the subject 5 with his or her exercise by applying at least one of a mechanical stimulus or an electrical stimulus to the subject 5. In this embodiment, the rehabilitation support system 100 is used to support the subject 5 in his or her rehabilitation to recover the function of his or her left hand fingers, and therefore, the exercise assisting device 3 is worn by the subject 5 on his or her left hand as shown in FIG. 1.

In the following description of this embodiment, the rehabilitation support system 100 is supposed to be used, for example, to support the subject 5 in his or her rehabilitation to recover the function of gripping something with his or her left hand fingers and the function of stretching his or her left hand fingers. As used herein, the "gripping action" refers to the action of gripping something. That is to say, in the case of this subject 5, his or her left hand fingers are the affected region, and the rehabilitation support system 100 is used to support the subject 5 in his or her rehabilitation to recover the ability to do voluntary movement, namely, the ability to grip something with his or her left hand fingers and the ability to stretch his or her left hand fingers. Actually, however, the rehabilitation support system 100 does not directly support the subject 5 in his or her gripping action but indirectly supports him or her in the attempt (rehabilitation) to recover the ability to grip something by assisting the subject 5 with his or her action of stretching hand fingers. As used herein, the "stretching action" refers to the action of opening a hand by stretching four hand fingers 53 (namely, second to fifth hand fingers), except the first hand finger (i.e., the thumb), or the action of releasing an "object" that the subject 5 is gripping through the gripping action.

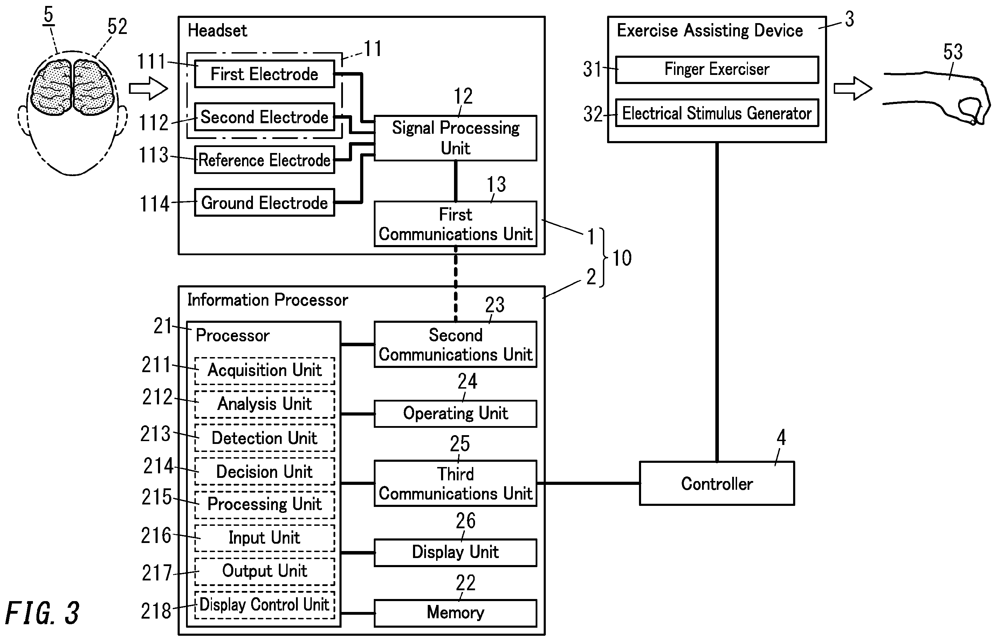

Thus, the rehabilitation support system 100 supports the subject 5, who is doing the stretching action as the voluntary movement, in his or her voluntary movement by making the exercise assisting device 3 worn by the subject 5 on his or her left hand apply at least one of a mechanical stimulus or an electrical stimulus to the subject's 5 left hand fingers 53. Specifically, the exercise assisting device 3 includes a finger exerciser 31 and an electrical stimulus generator 32 as shown in FIG. 3.

The finger exerciser 31 is a device for moving four hand fingers 53 (namely, the second to fifth hand fingers), except the first hand finger (thumb), by holding the four hand fingers 53 and applying a mechanical stimulus (external force) to these four hand fingers 53. The finger exerciser 31 includes a power source such a motor or a solenoid, and is configured to move the four hand fingers 53 by transmitting the force generated by the power source to the four hand fingers 53. The finger exerciser 31 is able to do two types of operations, namely, an "opening operation" of moving the four hand fingers 53 held away from the first finger (i.e., stretching the four hand fingers 53) and a "closing operation" of moving the four hand fingers 53 toward the first finger (i.e., making the hand fingers 53 grip something). The finger exerciser's 31 opening operation assists the subject 5 with his or her stretching action, and the finger exerciser's 31 closing operation assists the subject 5 with his or her gripping action.

The electrical stimulus generator 32 is a device for applying an electrical stimulus to the subject's 5 region for moving his or her hand fingers 53. In this case, the subject's 5 region for moving his or her hand fingers 53 includes a region corresponding to at least one of a muscle or a nerve of the subject's 5 hand fingers 53. For example, the subject's 5 region for moving his or her hand fingers 53 may be a part of the subject's 5 right or left arm. The electrical stimulus generator 32 includes a pad to be attached to the subject's 5 body (such as his or her right or left arm). The electrical stimulus generator 32 applies a stimulus to the region for moving the hand fingers 53 by applying an electrical stimulus (in the form of an electrical current) from the pad to the subject's 5 body.

The controller 4 controls the exercise assisting device 3 in accordance with the electroencephalogram information acquired by the electroencephalogram measurement system 10. In this embodiment, the controller 4 is electrically connected to the information processor 2 of the electroencephalogram measurement system 10 and the exercise assisting device 3. A power cable for supplying operating power to the exercise assisting device 3 and the controller 4 is connected to the controller 4. The controller 4 includes a driver circuit for driving the finger exerciser 31 of the exercise assisting device 3 and an oscillator circuit for driving the electrical stimulus generator 32. The controller 4 receives a control signal from the information processor 2 via wired communication, for example.

On receiving a first control signal from the information processor 2, the controller 4 makes its driver circuit drive the finger exerciser 31 of the exercise assisting device 3, thereby controlling the exercise assisting device 3 such that the finger exerciser 31 performs the "opening operation." Also, on receiving a second control signal from the information processor 2, the controller 4 makes its driver circuit drive the finger exerciser 31 of the exercise assisting device 3, thereby controlling the exercise assisting device 3 such that the finger exerciser 31 performs the "closing operation." Furthermore, on receiving a third control signal from the information processor 2, the controller 4 makes its oscillator circuit drive the electrical stimulus generator 32 of the exercise assisting device 3, thereby controlling the exercise assisting device 3 such that an electrical stimulus is applied to the subject's 5 body.

This allows the controller 4 to control the exercise assisting device 3 based on the electroencephalogram information acquired by the electroencephalogram measurement system 10 by controlling the exercise assisting device 3 in accordance with the control signals supplied from the electroencephalogram measurement system 10. Optionally, the controller 4 may also control the exercise assisting device 3 such that the finger exerciser 31 performs the "opening operation" and the "closing operation" in response to a turn of an operating switch provided for the controller 4.

Next, it will be described how to use this rehabilitation support system 100. In the following description of this embodiment, it will be described how the rehabilitation support system 100 supports the subject 5 in his or her voluntary movement (i.e., the stretching action) to be done by the subject 5 in order to release a peg 101 (see FIG. 1) from his or her left hand by stretching the hand fingers 53 from a position where he or she is gripping the peg 101 with his or her left hand fingers.

First, as a preparation process, the subject 5 wears the headset 1 on the head 52 and also wears the exercise assisting device 3 on his or her left hand. In this case, the subject 5 wears the headset 1 on his or her head 52 such that at least the electrode unit 11 is brought into contact with a part of the right surface of the subject's 5 head 52, which constitutes the region of interest 51. The exercise assisting device 3 is worn by the subject 5 so as to hold at least the four hand fingers 53 (i.e., the second to fifth hand fingers), except the first finger (thumb), of the subject's 5 left hand and attach the pad to the subject's 5 left arm. The headset 1 and the exercise assisting device 3 may be firmly fixed as appropriate so as not to be displaced or come loose during the rehabilitation. In the preparation process, the subject's 5 four hand fingers 53 are held by the finger exerciser 31 of the exercise assisting device 3 to make the subject 5 keep gripping the peg 101 with his or her left hand fingers. The subject 5 may be equipped with the headset 1 and the exercise assisting device 3 by either the subject 5 him- or herself or a medical staff.

When the preparation is done to make the headset 1 and the information processor 2 ready to communicate with each other, the electroencephalogram information generated by the headset 1 may be acquired by the information processor 2. That is to say, the electroencephalogram measurement system 10 may acquire, at the information processor 2, the electroencephalogram information representing an electroencephalogram obtained by the electrode unit 11 placed on the region of interest 51 that forms part of the subject's 5 head 52. As will be described in detail later in the "(3) Electroencephalogram measurement system" section, the information processor 2 makes its memory 22 (see FIG. 3) store (accumulate), along the time axis, the electroencephalogram information acquired. In addition, the information processor 2 generates a power spectrum of the electroencephalogram by carrying out a time frequency analysis on the electroencephalogram information stored, for example. This allows the electroencephalogram measurement system 10 to detect an electroencephalogram with a characteristic variation that may arise when the subject 5 plans to do voluntary movement by making the information processor 2 constantly monitor the data of the power spectrum.

In this case, before the subject 5 starts his or her rehabilitation, the electroencephalogram measurement system 10 performs calibration processing for determining various types of parameters for use to detect an electroencephalogram as the target of detection. This allows the electroencephalogram measurement system 10 to improve the accuracy of detecting the electroencephalogram as the target of detection with variations from one subject 5 to another, in, for example, the frequency band where the power declines due to the event-related desynchronization and the magnitude of the decline in power, taken into account. The calibration processing will be described in further detail later in the "(3) Electroencephalogram measurement system" section.

After having finished the preparation process including the calibration processing, the rehabilitation support system 100 starts performing a training process for supporting the subject 5 in his or her rehabilitation. In the training process, the subject 5 is supported in his or her rehabilitation based on the electroencephalogram measured by the electroencephalogram measurement system 10 during a training period. Specifically, the training period is subdivided into two periods, namely, a rest period and an exercise period. In each of the rest period and the exercise period, the subject 5 undergoes his or her rehabilitation in accordance with the instructions given by the rehabilitation support system 100. In this embodiment, the training period may be 10 seconds, and if the training period is evenly divided into two, then the first half of 5 seconds is supposed to be the "rest period" and the second half of 5 seconds is supposed to be the "exercise period."

In the rest period, the subject 5 puts his or her body at rest (i.e., does not plan to do (or imagine doing) any voluntary movement) to keep relaxed. At this time, the electroencephalogram measurement system 10 does not detect any electroencephalogram with a characteristic variation that may arise due to the event-related desynchronization when the subject 5 plans to do the voluntary movement.

Meanwhile, in the exercise period, the subject 5 plans to do (or imagines doing) the action of stretching the hand fingers 53 as a type of voluntary movement. At this time, the electroencephalogram measurement system 10 may detect an electroencephalogram with a characteristic variation that may arise due to the event-related desynchronization when the subject 5 plans to do the voluntary movement. In this embodiment, such a characteristic variation in electroencephalogram is detected by comparing an activation level with a threshold value and determining whether or not the activation level is greater than the threshold value. As used herein, the "activation level" refers to a value representing the magnitude of decline in power (i.e., power spectrum) in a particular frequency band. When the event-related desynchronization causes a decline in the power in the particular frequency band, the activation level exceeds the threshold value. Thus, the electroencephalogram measurement system 10 detects the characteristic variation in electroencephalogram when finding the activation level greater than the threshold value.

In this electroencephalogram measurement system 10, generation of a control signal for controlling the exercise assisting device 3 is triggered by the detection of the electroencephalogram with such a characteristic variation. This allows, when the subject 5 plans to do voluntary movement, the rehabilitation support system 100 to make the exercise assisting device 3 assist the subject 5 with the voluntary movement at the timing when a brain region, corresponding to the target region of the voluntary movement, is actually activated.

The operation of the electroencephalogram measurement system 10 during the training process will be described in further detail later in the "(3) Electroencephalogram measurement system" section.

### (3) Electroencephalogram measurement system

### (3.1) Configuration

The electroencephalogram measurement system 10 according to this embodiment will be described in further detail.

As shown in FIG. 3, the electroencephalogram measurement system 10 includes: the headset 1 to be worn by the subject 5 on his or her head 52; and the information processor 2 including, as a major constituent element, a computer system such as a personal computer.

The headset 1 includes: the electrode unit 11; a signal processing unit 12; and a first communications unit 13. The headset 1 may be driven by a battery, for example, which supplies power for operating the signal processing unit 12 and the first communications unit 13.

The electrode unit 11 includes electrodes for measuring and recording the subject's 5 electroencephalogram (in the form of an electroencephalogram signal), which may be silver-silver chloride electrodes, for example. The electrode unit 11 may also be made of gold, silver, or platinum, for example. The electrode unit 11 includes a first electrode 111 and a second electrode 112. In this embodiment, the region of interest 51 set on the surface of the subject's 5 head 52 includes a first region of interest 511 and a second region of interest 512 as shown in FIG. 2. The first electrode 111 corresponds to, and is placed on, the first region of interest 511. The second electrode 112 corresponds to, and is placed on, the second region of interest 512. Specifically, the first region of interest 511 and the second region of interest 512 are arranged in this order on a line segment connecting the median center of the head 52 to the subject's 5 right ear such that the first region of interest 511 is located closer to the median center than (i.e., located over) the second region of interest 512 is.

Also, in this embodiment, the headset 1 further includes a reference electrode 113 and a ground electrode 114. The reference electrode 113 is an electrode for measuring the reference potential of the electroencephalogram signal to be measured by each of the first electrode 111 and the second electrode 112. The reference electrode 113 is arranged behind either the right ear or the left ear of the head 52. Specifically, the reference electrode 113 is arranged behind the ear, on which the first electrode 111 and the second electrode 112 are placed, on the head 52. In this embodiment, the first electrode 111 and the second electrode 112 are placed on the right surface of the head 52, and therefore, the reference electrode 113 is arranged behind the right ear. On the other hand, the ground electrode 114 is arranged behind the right or left ear, on which the reference electrode 113 is not placed, on the head 52. In this embodiment, the reference electrode 113 is arranged behind on the right ear, and therefore, the ground electrode 114 is arranged behind the left ear. Each of the reference electrode 113 and the ground electrode 114 is electrically connected to the body 15 of the headset 1 via electric wires 16 (see FIG. 2) and is attached to the surface of the head 52 (i.e., the scalp). Optionally, the reference electrode 113 and the ground electrode 114 do not have to be placed behind the right or left ear but may also be placed on the right or left earlobe. In any case, the region behind the right or left ear and the right or left earlobe are regions of the head that are not easily affected by a bioelectric potential deriving from a brain activity. In other words, the reference electrode 113 and the ground electrode 114 are suitably arranged in such regions of the head that are not easily affected by a bioelectric potential deriving from the brain activity.

The signal processing unit 12 is electrically connected to the electrode unit 11, the reference electrode 113, and the ground electrode 114 to perform signal processing on the electroencephalogram signal (electrical signal) supplied from the electrode unit 11 and generate electroencephalogram information. That is to say, the headset 1 detects the subject's 5 electroencephalogram by having the electrode unit 11 measure the activity potential of the subject's 5 brain, to make the signal processing unit 12 generate electroencephalogram information representing the electroencephalogram. The signal processing unit 12 includes at least an amplifier for amplifying the electroencephalogram signal and an A/D converter for converting an analog electroencephalogram signal into a digital signal, and outputs, as the electroencephalogram information, the amplified digital electroencephalogram signal.

The first communications unit 13 has the capability of communicating with the information processor 2. The first communications unit 13 transmits, to the information processor 2, at least the electroencephalogram information generated by the signal processing unit 12. In this embodiment, the first communications unit 13 is able to communicate with the information processor 2 bidirectionally. The communications protocol of the first communications unit 13 may be wireless communication compliant with the Bluetooth^{®} standard. The electroencephalogram information is transmitted, as needed, from the first communications unit 13 to the information processor 2.

The information processor 2 includes, as its major constituent element, a computer system including a processor 21 and a memory 22. The information processor 2 further includes a second communications unit 23, an operating unit 24, a third communications unit 25, and a display unit 26.

The second communications unit 23 has the capability of communicating with (the first communications unit 13 of) the headset 1. The second communications unit 23 receives at least the electroencephalogram information from the headset 1. In this embodiment, the second communications unit 23 is able to communicate with the headset 1 bidirectionally. The second communications unit 23 receives, as needed, the electroencephalogram information, sampled at a sample frequency of about 200 Hz, for example, from the headset 1.

The third communications unit 25 has the capability of communicating with the controller 4. The third communications unit 25 transmits at least a control signal to the controller 4. The communications protocol of the third communications unit 25 is a wired communication compliant with the universal serial bus (USB) standard.

In this embodiment, the information processor 2 is equipped with a touchscreen panel display, which functions as the operating unit 24 and the display unit 26. Thus, the information processor 2 determines, when the operating unit 24 detects any of various types of operations (including tapping, swiping, and dragging) on buttons and other objects on the screen of the display unit 26, that the buttons and other objects should have been operated. That is to say, the operating unit 24 and the display unit 26 function as a user interface that not only displays various types of information thereon but also accepts operating commands entered by either the subject 5 or a medical staff. Note that the operating unit 24 does not have to be implemented as a touchscreen panel display but may also be implemented as a keyboard, a pointing device, a mechanical switch, or any other type of input device.

The processor 21 has various functions serving as an acquisition unit 211, an analysis unit 212, a detection unit 213, a decision unit 214, a processing unit 215, an input unit 216, an output unit 217, and a display control unit 218. The respective functions of the acquisition unit 211, analysis unit 212, detection unit 213, decision unit 214, processing unit 215, input unit 216, output unit 217, and display control unit 218 are performed by having a program stored in the memory 22 executed by the processor 21.

The acquisition unit 211 acquires electroencephalogram information representing an electroencephalogram obtained by the electrode unit 11 placed on the region of interest 51 that forms part of the subject's 5 head 52. That is to say, the acquisition unit 211 acquires, from the headset 1 and via the second communications unit 23, the electroencephalogram information representing the electroencephalogram obtained by the electrode unit 11 of the headset 1. Specifically, in this embodiment, the acquisition unit 211 acquires first electroencephalogram information representing the electroencephalogram obtained by the first electrode 111 and second electroencephalogram information representing the electroencephalogram obtained by the second electrode 112. That is to say, in this embodiment, the electrode unit 11 includes the first electrode 111 and the second electrode 112. Thus, the acquisition unit 211 regards the electroencephalogram information collected by the first electrode 111 and the electroencephalogram information collected by the second electrode 112 as the first electroencephalogram information and the second electroencephalogram information, respectively. In this embodiment, the acquisition unit 211 acquires electroencephalogram information in a digital form and stores, in the memory 22, the electroencephalogram information thus acquired. At this time, the time-series data of the electroencephalogram information collected by the electroencephalogram measurement system 10 since the beginning through the end of the training period is stored in the memory 22.

The analysis unit 212 analyzes the electroencephalogram information acquired by the acquisition unit 211. The analysis unit 212 performs a frequency analysis on the electroencephalogram information stored in the memory 22, thereby generating spectrum data representing the signal intensity on a frequency band basis. Specifically, the analysis unit 212 reads pieces of the electroencephalogram information, corresponding to a predetermined period of time, from the memory 22 and carries out a frequency analysis such as a short-time Fourier transform (STFT) on the pieces of the electroencephalogram information. This allows the power to be calculated on a frequency band basis with respect to the electroencephalogram signal that varies with time. As used herein, the "power" refers to an integrated value of the intensities on a frequency band basis (i.e., spectrum intensities).

The detection unit 213 detects, based on the power on the frequency band basis that has been analyzed by the analysis unit 212, an electroencephalogram with a characteristic variation that may arise when the subject 5 plans to do the voluntary movement. Specifically, the detection unit 213 detects either the presence or absence of an electroencephalogram with the characteristic variation by determining whether the power in the particular frequency band falls within a rest range or an exercise range. As used herein, the "rest range" refers to a range in which the power falling within the particular frequency band of the subject's 5 electroencephalogram may take when the subject 5 puts his or her body at rest, i.e., plans to do (or imagines doing) no voluntary movement and keeps relaxed. On the other hand, the "exercise range" as used herein refers to a range in which the power falling within the particular frequency band of the electroencephalogram may take when the subject 5 plans to do (or imagines doing) the voluntary movement. That is to say, the detection unit 213 determines, when finding that the power falling within the particular frequency band has made a transition from the rest range to the exercise range, that an electroencephalogram with a characteristic variation that may arise when the subject 5 plans to do the voluntary movement should have been produced.

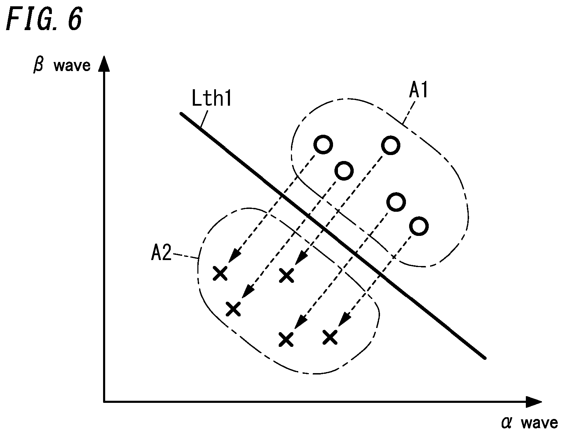

More specifically, according to this embodiment, the detection unit 213 may calculate, based on the power on the frequency band basis analyzed by the analysis unit 212, an activation level indicating the magnitude of decline in power in the particular frequency band, for example. Then, the detection unit 213 compares the activation level calculated with a threshold value stored in the memory 22, and determines, when finding the activation level greater than the threshold value, that the characteristic variation should have arisen in the electroencephalogram. That is to say, the threshold value with respect to the activation level is a value, in a graph representing the power in the particular frequency band (see FIG. 6), corresponding to the border line (or the line Lth1) between the rest range A1 and the exercise range A2. Thus, when the event-related desynchronization causes a decline in the power of the particular frequency band to have the power falling within the particular frequency band make a transition from the rest range to the exercise range, the activation level exceeds the threshold value. As will be described in detail later in the "(3.3) Calibration processing" section, the threshold value is set through the calibration processing.

The decision unit 214 determines, based on the electroencephalogram information acquired by the acquisition unit 211, whether or not there are any artifacts. The artifacts may constitute noise components with respect to the electroencephalogram as described above. That is why if the decision unit 214 determines that there should be some artifacts, then the electroencephalogram measurement accuracy may cause a decline. The operation of the decision unit 214 will be described in further detail later in the "(3.3) Calibration processing" section.

The processing unit 215 carries out the calibration processing to determine the parameters for use to analyze the electroencephalogram information. That is to say, the processing unit 215 carries out the calibration processing to determine various types of parameters including at least the threshold value with respect to the activation level. The calibration processing is carried out by the processing unit 215 before the training process is started. The processing unit 215 stores, in the memory 22, various types of parameters determined through the calibration processing.

The input unit 216 accepts, from the operating unit 24, a designation signal designating one or more sets of electroencephalogram information for use in the calibration processing, from among multiple sets of electroencephalogram information acquired by the acquisition unit 211. That is to say, the memory 22 may store multiple sets of electroencephalogram information acquired by the acquisition unit 211. In the calibration processing, one or more sets of electroencephalogram information, designated by the designation signal, is used selectively from among those multiple sets of electroencephalogram information. In other words, the designation signal that the input unit 216 accepts from the operating unit 24 is a signal for designating the one or more sets of electroencephalogram information for use in the calibration processing, from among the multiple sets of electroencephalogram information stored in the memory 22.

The output unit 217 outputs the decision made by the decision unit 214. That is to say, when the decision unit 214 has made a decision about the presence or absence of any artifacts, the decision is output from the output unit 217. The decision may be output from the output unit 217 as a text message displayed, a verbal message emitted, a page printed out, a file written into a non-transitory storage medium, or data transmitted to a telecommunications device, for example. In this embodiment, the output unit 217 outputs the decision to the display control unit 218 such that the decision is displayed as a message on the display unit 26.

The display control unit 218 makes the display unit 26 display the decision output from the output unit 217. The display control unit 218 has the capability of controlling the display unit 26 and may have various types of content displayed on the display unit 26. The display control unit 218 has at least the decision made by the decision unit 214 displayed on the display unit 26.

### (3.2) Training process

Next, it will be described in detail how the electroencephalogram measurement system 10 operates during the training process.

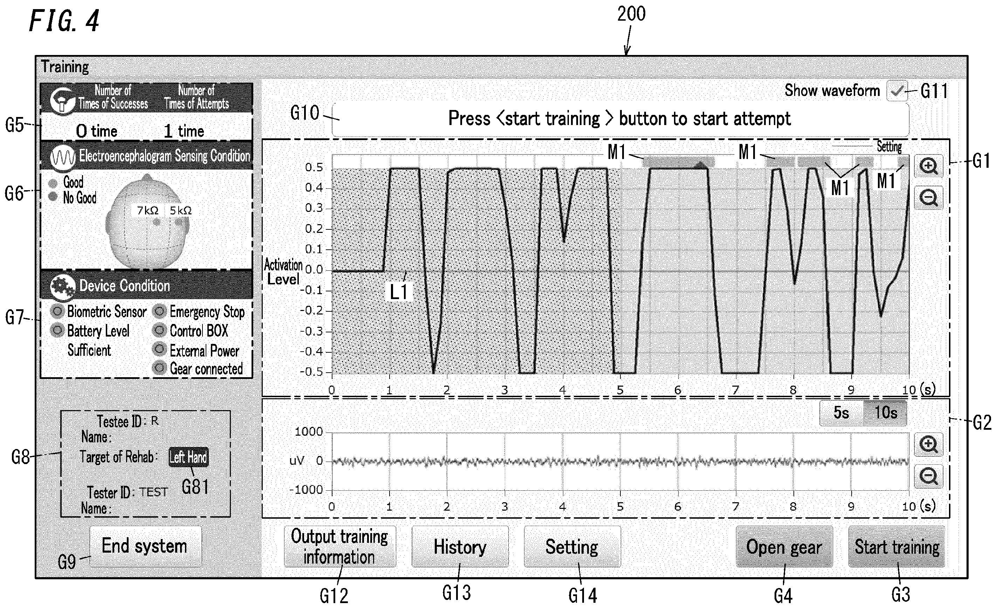

During the training process, the operation mode of the rehabilitation support system 100 is a training mode. When the operation mode of the rehabilitation support system 100 is the training mode, a training screen 200 such as the one shown in FIG. 4 is displayed on the display unit 26 of the information processor 2. Note that in the example illustrated in FIG. 4, the one-dot chain indicating the region and the reference signs are just shown there for the sake of convenience and are actually not displayed on the display unit 26.

The training screen 200 includes an activation level display area G1, an electroencephalogram display area G2, a start training button G3, a gear operating button G4, a number of times display area G5, a sensing condition display area G6, a device condition display area G7, a status display area G8, and an end button G9. The training screen 200 further includes an operation guidance area G10, a checkbox G11, a output training information button G12, a history button G13, and a setting button G14.

The activation level display area G1 is an area for displaying an activation level graph. The activation level graph, of which the abscissa indicates the time (in seconds) and the ordinate indicates the activation level, displays a waveform showing how the activation level changes with time. In the activation level display area G1, the line L1 indicates a threshold value (as a setting). Also, in a period during which the activation level is greater than the threshold value, a decision mark M1 in the shape of a band is displayed over the activation level graph. In addition, the activation level graph suitably has two different background colors for the rest period (i.e., a period from 0 to 5 seconds) defining the first half of the training period and the exercise period (i.e., a period from 5 to 10 seconds) defining the second half of the training period, respectively.

The electroencephalogram display area G2 is an area for displaying the electroencephalogram measured by the headset 1. The electroencephalogram is displayed in the electroencephalogram display area G2 in real time in accordance with the electroencephalogram information transmitted from the headset 1 to the information processor 2. The electroencephalogram graph, of which the abscissa indicates the time (in seconds) and the ordinate indicates the potential, displays a waveform indicating how the electroencephalogram changes with time.

The start training button G3 is a button to be tapped to get the training started. Tapping the start training button G3 causes the training by the rehabilitation support system 100 to be started and also causes the electroencephalogram measurement system 10 to start measuring the electroencephalogram.

The gear operating button G4 is a button allowing the subject 5 to operate the exercise assisting device 3. Tapping the gear operating button G4 causes the electroencephalogram measurement system 10 to output a control signal to have the operation of closing or opening the exercise assisting device 3 performed. In this example, text information such as "close gear" or "open gear" is displayed on the gear operating button G4. Tapping the gear operating button G4 with "close gear" displayed causes the operation of closing the exercise assisting device 3 to be performed. Tapping the gear operating button G4 with "open gear" displayed causes the operation of opening the exercise assisting device 3 to be performed.

The number of times display area G5 is an area for displaying the number of times of successes and the number of times of attempts. As used herein, the "number of times of successes" indicates the number of times the electroencephalogram measurement system 10 has successfully detected the electroencephalogram, having the characteristic variation that may arise due to event-related desynchronization when the subject 5 plans to do the voluntary movement, during the exercise period of the training period. Also, as used herein, the "number of times of attempts" indicates the number of times the training by the rehabilitation support system 100 has been carried out.

The sensing condition display area G6 is an area for displaying a specific condition in which the electroencephalogram is sensed. In this embodiment, the electroencephalogram measurement system 10 measures the impedance value between the first electrode 111 and the subject's 5 body (i.e., his or her scalp) and the impedance value between the second electrode 112 and the subject's 5 body (i.e., his or her scalp), thereby determining, based on the impedance values measured, whether the electroencephalogram is sensed in good condition or not. In the example illustrated in FIG. 4, in the sensing condition display area G6, displayed are a text indicating the impedance values measured and markers indicating whether the electroencephalogram is sensed by each of the first electrode 111 and the second electrode 112 in good condition or not. Specifically, the markers on the illustration representing a human head change their colors depending on whether the electroencephalogram is sensed by each of the first electrode 111 and the second electrode 112 in good condition or not.

The device condition display area G7 is an area for displaying the respective conditions of various devices that form the rehabilitation support system 100. In the device condition display area G7, the condition of connection between the information processor 2 and the headset 1, the battery level of the headset 1, and other conditions are indicated by icons such as open circles (O) and crosses (X).

The status display area G8 is an area for displaying the identification information (testee ID) and name of the subject 5, and the identification information (tester ID) and name of the medical staff attending him or her. In addition, in the status display area G8, a target icon G81, indicating whether the "target of rehabilitation" is "left hand" or "right hand" is further displayed. In the example illustrated in FIG. 4, the target icon G81 indicates, as text information, that the target of rehabilitation is the "left hand."

The end button G9 is a button to be tapped to end the training. Tapping the end button G9 brings not only the training by the rehabilitation support system 100 but also the electroencephalogram measurement by the electroencephalogram measurement system 10 to an end.

The operation guidance area G10 is an area for displaying text information providing guidance on how to operate the rehabilitation support system 100. In the example illustrated in FIG. 4, the text information that says "press <start training > button to start attempt" is displayed in the operation guidance area G10. The message displayed in the operation guidance area G10 varies according to the operating state of the rehabilitation support system 100.

The checkbox G11 is an icon for switching the state of displaying the electroencephalogram in the electroencephalogram display area G2 into the state of displaying no electroencephalograms there, and vice versa. Tapping the checkbox G11 causes the display state to be switched alternately from the state where the electroencephalogram is displayed in the electroencephalogram display area G2 to the state where the electroencephalogram is not displayed in the electroencephalogram display area G2, and vice versa. While the checkbox G11 is checked off, the electroencephalogram is displayed in the electroencephalogram display area G2.

The output training information button G12 is a button for outputting training information including the result of rehabilitation. Tapping the output training information button G12 causes the training information to be output in any desired form. For example, the training information may be output as a text message displayed, a verbal message emitted, a page printed out, a file written into a non-transitory storage medium, or data transmitted to a telecommunications device, for example.

The history button G13 is a button allowing the user to make reference to the history of the training information including the results of rehabilitation. Tapping the history button G13 changes the screens displayed on the display unit 26 of the information processor 2 from the training screen 200 to a history reference screen. On the history reference screen, at least the results of rehabilitations that the subject 5 has undergone so far are displayed.

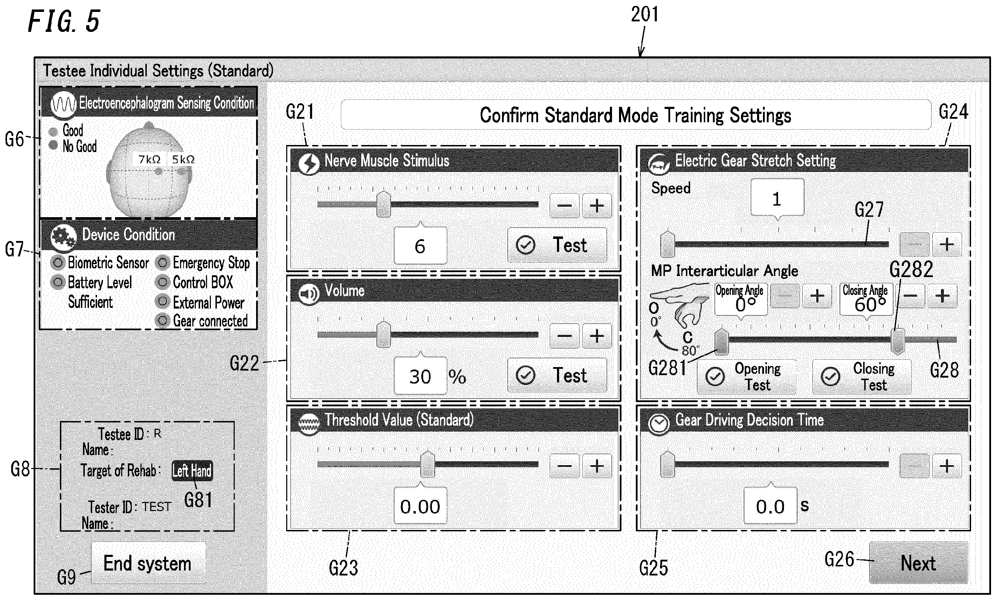

The setting button G14 is a button for changing the operation mode of the rehabilitation support system 100 into the setting mode in which various types of settings are made about the rehabilitation support system 100. Tapping the setting button G14 changes the screens displayed on the display unit 26 of the information processor 2 from the training screen 200 to a setting screen 201 to be described later (see FIG. 5).

During the training process, the subject 5 undergoes the rehabilitation with the training screen 200 described above displayed on the display unit 26 of the information processor 2. That is to say, tapping the start training button G3 causes the training by the rehabilitation support system 100 to be started.

As soon as the training is started, the training time starts being counted, and the electroencephalogram measurement system 10 starts measuring the electroencephalogram specific to the subject 5. During the rest period (i.e., the period from 0 to 5 seconds) that is the first half of the training period, the subject 5 puts his or her body at rest in accordance with the guidance displayed in the operation guidance area G10 or the instruction given by the medical staff attending him or her. At this time, the activation level and the electroencephalogram are displayed in real time on the training screen 200. During the rest period, however, the electroencephalogram measurement system 10 does not compare the activation level with the threshold value and does not detect an electroencephalogram with the characteristic variation caused by the event-related desynchronization, either.

On the other hand, during the exercise period (i.e., the period from 5 to 10 seconds) defining the second half of the training period, the subject 5 plans to do (or imagines doing) the action of stretching his or her hand fingers 53 as a voluntary movement in accordance with either the guidance displayed in the operation guidance area G10 or the instruction given by the medical staff attending him or her. At this time, the activation level and the electroencephalogram are displayed in real time on the training screen 200. In addition, during the exercise period, the electroencephalogram measurement system 10 compares the activation level with the threshold value, thereby detecting an electroencephalogram with the characteristic variation caused by the event-related desynchronization. In this case, when the event-related desynchronization causes the activation level to exceed the threshold value (as indicated by the line L1 in FIG. 4), the band-shaped decision mark M1 appears over the activation level graph on the training screen 200.

More specifically, the electroencephalogram measurement system 10 makes the analysis unit 212 analyze, as needed, the electroencephalogram information acquired by the acquisition unit 211. In addition, the electroencephalogram measurement system 10 makes the detection unit 213 calculate the activation level based on the power on the frequency band basis analyzed by the analysis unit 212 and compare the activation level with the threshold value. The electroencephalogram measurement system 10 determines, when the event-related desynchronization causes a decline in the power in the particular frequency band to change the power in the particular frequency band from the rest range to the exercise range, that the activation level should have exceeded the threshold value.

In this case, the particular frequency band may be either a single frequency band (which may be either an α-wave range or a β-wave range) or a plurality of frequency bands (which may be both the α-wave range and the β-wave range). In a situation where the particular frequency band includes two frequency bands, for example, when the coordinate values defined by the powers of these two frequency bands make a transition from the rest range to the exercise range, the activation level exceeds the threshold value.

In addition, according to this embodiment, the electroencephalogram measurement system 10 also has the capability of measuring the duration for which the activation level remains greater than the threshold value. In the training screen 200 illustrated as an example in FIG. 4, the length of each decision mark M1 corresponds to the length of the duration. When the activation level rises (i.e., changes) from a value equal to or less than the threshold value to a value greater than the threshold value, the electroencephalogram measurement system 10 transmits the third control signal to the exercise assisting device 3. Furthermore, when the duration reaches a prescribed amount of time (of 1 second, for example), the electroencephalogram measurement system 10 transmits the first control signal to the exercise assisting device 3.

As can be seen from the foregoing description, when the activation level exceeds the threshold value, the electrical stimulus generator 32 of the exercise assisting device 3 is driven and the exercise assisting device 3 applies an electrical stimulus to the subject's 5 body, thereby assisting the subject 5 with his or her voluntary movement (i.e., the stretching action in this case). Furthermore, when the activation level remains greater than the threshold value for the prescribed amount of time, the finger exerciser 31 of the exercise assisting device 3 is driven and the exercise assisting device 3 performs the "opening operation" with respect to the finger exerciser 31, thereby assisting the subject 5 with his or her voluntary movement (stretching action in this case).

As a result, in a situation where the subject 5 plans to do (or imagines doing) the voluntary movement, the exercise assisting device 3 assists the subject 5 with his or her voluntary movement (i.e., the action of stretching his or her left hand fingers 53) at the timing when a brain region corresponding to the target region of the voluntary movement is actually activated. At this time, the subject's 5 muscle and sensory nerve start their activity and the information is transmitted to the brain, thereby reconstructing the nerve system and achieving some rehabilitation effect. This allows the rehabilitation support system 100 to support the subject 5 in his or her rehabilitation by exercise therapy as well as in a situation where medical staff assists subjects 5 in various conditions and more effectively than in a situation where the subject 5 does the voluntary movement by him- or herself.

Meanwhile, when the operation mode of the rehabilitation support system 100 is the setting mode, a setting screen 201 such as the one shown in FIG. 5 is displayed on the display unit 26 of the information processor 2. Note that in the example illustrated in FIG. 5, the one-dot chain indicating the region and the reference signs are just shown there for the sake of convenience and are actually not displayed on the display unit 26.

The setting screen 201 includes, as well as the training screen 200, the sensing condition display area G6, the device condition display area G7, the status display area G8, and the end button G9 but also first to fifth setting areas G21-G25 and an update button G26 as well.

In the first setting area G21, a slide bar for adjusting the intensity of the electrical stimulus applied by the electrical stimulus generator 32 (i.e., the nerve muscle stimulus level) is displayed. In the second setting area G22, a slide bar for controlling the volume is displayed. In the third setting area G23, a slide bar for adjusting the threshold value with respect to the activation level is displayed. In the fourth setting area G24, an upper slide bar G27 for controlling the operating velocity of the finger exerciser 31 and a lower slide bar G28 for adjusting the movable range of the finger exerciser 31 are displayed.

In the fifth setting area G25, a slide bar for setting a prescribed time is displayed. As used herein, the "prescribed time" refers to an amount of time for determining whether or not to assist the subject 5 with his or her stretching action through the opening operation of the finger exerciser 31 and is set at 1.0 second by default. When finding the activation level remaining greater than the threshold value for the prescribed time, the electroencephalogram measurement system 10 outputs the first control signal to drive the finger exerciser 31.

In this case, as for the movable range of the finger exerciser 31, an "opening angle" defining the end point of the opening operation and a "closing angle" defining the end point of the closing operation may be set on an individual basis. Specifically, the lower slide bar G28 includes a first slider G281 for adjusting the opening angle and a second slider G282 for adjusting the closing angle. Furthermore, to allow the subject 5 to control the movable range of the finger exerciser 31 intuitively, the first slider G281, the illustration representing the action of the opening operation, and the text information indicating the opening angle are displayed in the same color (such as in light blue). Likewise, the second slider G282, the illustration representing the action of the closing operation, and the text information indicating the closing angle are displayed in the same color (such as in red).

The update button G26 is a button for changing the operation mode of the rehabilitation support system 100 into the training mode. Tapping the update button G26 changes the screens displayed on the display unit 26 of the information processor 2 from the setting screen 201 into the training screen 200. At this time, various settings entered on the setting screen 201 are written into the memory 22 to update the settings.

### (3.3) Calibration processing

Next, the operation of the electroencephalogram measurement system 10 at the time of the calibration processing to be performed during the preparation process before the training process will be described in detail.

### (3.3.1) Overview of the processing

The calibration processing is performed when the operation mode of the rehabilitation support system 100 is a calibration mode. The calibration processing is processing for determining various types of parameters including at least the threshold value corresponding to the activation level. That is to say, the frequency band in which the power declines due to the event-related desynchronization and the magnitude of the decline in the power vary according to the attributes of the subject 5 (such as his or her age and sex), the affected region, the affected condition, and individual differences as described above. Thus, to improve the detection accuracy of the electroencephalogram to be detected, the electroencephalogram measurement system 10 determines, through the calibration processing, various types of parameters for use to analyze the electroencephalogram information for individual subjects 5.

The calibration processing includes: measurement processing for making the electroencephalogram measurement system 10 actually measure the subject's 5 electroencephalogram in the same procedure as in the training process; and calculation processing for determining various types of parameters according to this particular subject 5 based on the electroencephalogram measured.

In the measurement processing, a calibration period, which is divided into the rest period and the exercise period as in the training period, is set, and the electroencephalogram is measured by the electroencephalogram measurement system 10 during this calibration period. In each of the rest period and exercise period of the calibration period, the subject 5 undergoes his or her rehabilitation in accordance with the instructions given by the rehabilitation support system 100. In this embodiment, the calibration period may be 10 seconds, and if the calibration period is evenly divided into two, then the first half of 5 seconds is supposed to be the "rest period" and the second half of 5 seconds is supposed to be the "exercise period."

In the rest period, the subject 5 puts his or her body at rest (i.e., does not plan to do (or imagine doing) the voluntary movement) to keep relaxed. On the other hand, in the exercise period, the subject 5 plans to do (or imagines doing) the action of stretching his or her hand fingers 53 as a voluntary movement. In both of the rest and exercise periods, the electroencephalogram measurement system 10 measures the electroencephalogram specific to the subject 5. Also, the electroencephalogram measurement system 10 has the electroencephalogram information, measured during the calibration period (including the rest period and the exercise period), stored as a record in the memory 22. As used herein, the "record" refers to the time-series data of the electroencephalogram information measured by the electroencephalogram measurement system 10 since the beginning through the end of the calibration period during the measurement processing. Nevertheless, during the calibration period of the measurement processing, the electroencephalogram measurement system 10 does not perform the processing of detecting a characteristic variation in the electroencephalogram unlike during the training period.

In this case, the electroencephalogram measurement system 10 may perform the measurement processing a number of times during the calibration processing. The memory 22 may store a plurality of records as long as the number of records stored is equal to or less than a predetermined upper limit (of 30, for example). Thus, at the end point of the measurement processing that has been performed either once or a number of times, a single or a plurality of records, representing the subject's 5 electroencephalogram during the rest period and the exercise period, are stored in the memory 22.

The calculation processing includes determining various types of parameters on an individual subject 5 basis using the single or plurality of records stored in the memory 22 during the measurement processing that has been performed either once or a number of times. Specifically, the electroencephalogram measurement system 10 makes the analysis unit 212 analyze, as needed, the electroencephalogram information acquired by the analysis unit 212 to determine various types of parameters based on the power analyzed on a frequency band basis by the analysis unit 212. The parameters to be determined at this time include at least a threshold value with respect to the activation level and the respective frequency bands of the α and β waves.

Next, it will be described briefly with reference to FIG. 6 how to determine a threshold value with respect to a given activation level. FIG. 6 is a graph plotting respective powers in the rest period and the exercise period in a situation where attention is paid to two frequency bands of the α and β waves with respect to a plurality of (e.g., five in this example) records. In FIG. 6, the abscissa indicates the power of the α wave, the ordinate indicates the power of the β wave, representative values (such as average values) of the power during the rest period are indicated by open circles (O), and representative values (such as average values) of the power during the exercise period are indicated by crosses (X). In FIG. 6, the respective frequency bands of the α and β waves are supposed to have already been determined with respect to the subject 5.

That is to say, in FIG. 6, with respect to the two frequency bands of the α and β waves, the range with the open circles (O) corresponds to the rest range A1 and the range with the crosses (X) corresponds to the exercise range A2. As described above, the electroencephalogram measurement system 10 determines, when finding that the power falling within the particular frequency band has made a transition from the rest range to the exercise range, that an electroencephalogram with a characteristic variation that may arise when the subject 5 plans to do the voluntary movement should have been produced. Thus, the border line (i.e., the line Lth1) between the rest range A1 and the exercise range A2 in FIG. 6 corresponds to the threshold value with respect to the activation level. As can be seen, the threshold value with respect to the activation level may be determined based on a single or a plurality of records obtained during the measurement processing.

The various types of parameters determined through the calibration processing are stored in the memory 22. In the training process to be performed later, the rehabilitation support system 100 will use the various types of parameters determined through the calibration processing. Also, in the third setting area G23 of the setting screen 201 (see FIG. 5), the threshold value with respect to the activation level is adjustable with the slide bar as described above. Nevertheless, on the setting screen 201, the threshold value that has been determined through the calibration processing is just adjustable so as to be shifted in either the positive direction or the negative direction. That is to say, it still holds true that the threshold value as a reference value is determined through the calibration processing.

Note that the threshold value does not have to be determined by the method described above, but may also be determined by any of various other methods such as linear discriminant analysis (LDA) and support vector machine (SVM).

### (3.3.2) Description of screens

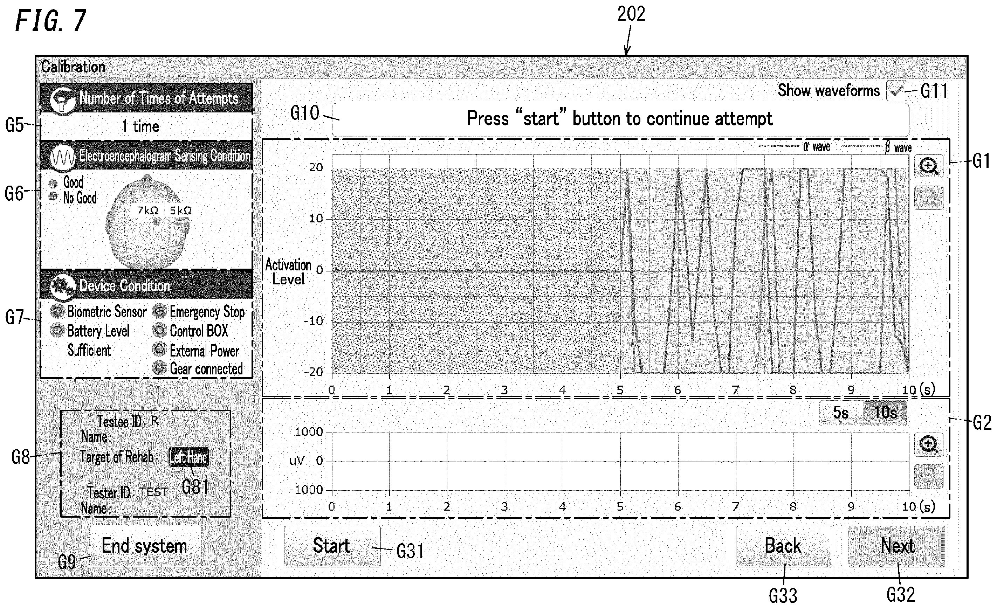

Next, the screens to be displayed on the display unit 26 of the information processor 2 during the calibration processing will be described with reference to FIGS. 7-9. Note that in the examples illustrated in FIGS. 7-9, the one-dot chain indicating the region and the reference signs are just shown there for the sake of convenience and are actually not displayed on the display unit 26.

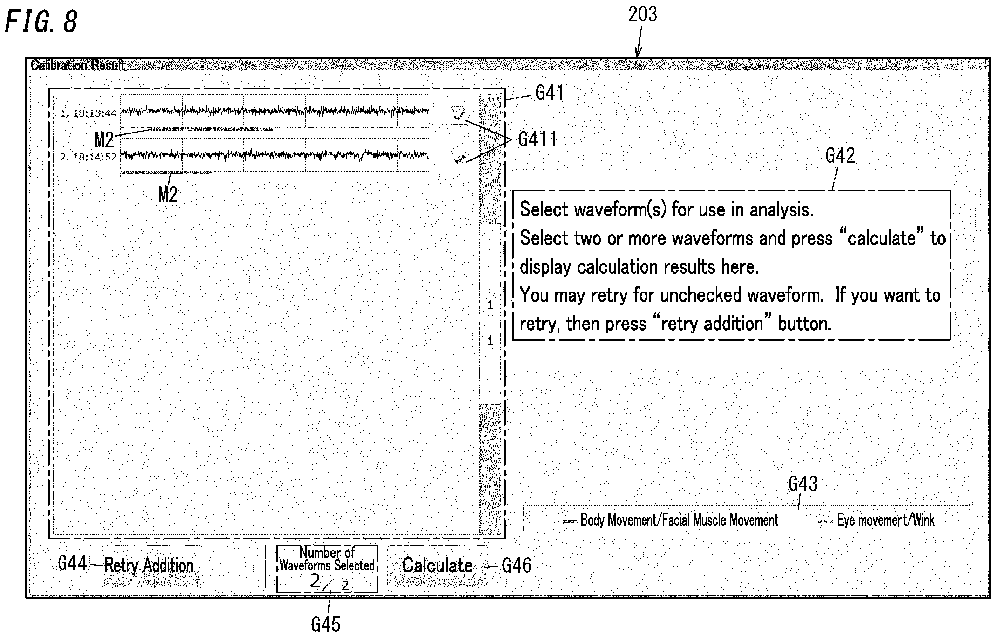

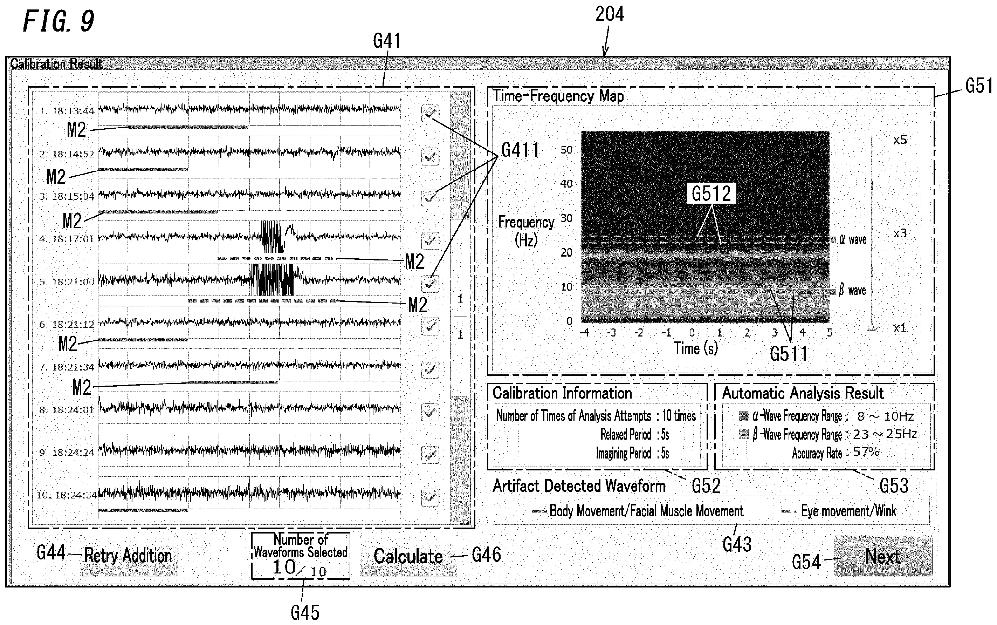

Specifically, first, during the measurement processing of the calibration processing, a calibration screen 202 such as the one shown in FIG. 7, for example, is displayed on the display unit 26 of the information processor 2. When the measurement processing ends, a selection screen 203 such as the one shown in FIG. 8 is displayed on the display unit 26 of the information processor 2. On the selection screen 203, a record for use in the calculation processing is selected. When the calculation processing ends, a calibration result screen 204 such as the one shown in FIG. 9 is displayed on the display unit 26 of the information processor 2.

As shown in FIG. 7, the calibration screen 202, as well as the training screen 200, also includes the activation level display area G1, the electroencephalogram display area G2, the number of times display area G5, the sensing condition display area G6, the device condition display area G7, the status display area G8, and the end button G9. The calibration screen 202 further includes not only the operation guidance area G10 and the checkbox G11 similar to their counterparts of the training screen 200, but also a start measurement button G31, a next button G32, and a back button G33 as well. The activation level display area G1 of the calibration screen 202 is basically the same as its counterpart of the training screen 200, but is different from the counterpart of the training screen 200 in that respective graphs of the α and β waves are displayed in the activation level display area G1. Likewise, the number of times display area G5 of the calibration screen 202 is basically the same as its counterpart of the training screen 200, but is different from the counterpart of the training screen 200 in that the "number of times of successes" is not displayed there.

In the activation level display area G1 of the calibration screen 202, a graph representing the activation level for the α wave (hereinafter referred to as an "α wave graph") and a graph representing the activation level for the β wave (hereinafter referred to as a "β wave graph") are displayed simultaneously. Nevertheless, the α wave graph and the β wave graph are displayed in mutually different modes (e.g., in two different colors) so as to be easily distinguished from each other. In addition, in the calibration processing, the threshold value with respect to the activation level has not been set yet. Thus, in the activation level display area G1, the line L1 indicating the threshold value (setting) is not displayed and no decision marks M1 are displayed, either, over the graph indicating the activation level. The activation level graph suitably has two different background colors for the rest period (i.e., the period of 0 to 5 seconds) defining the first half of the calibration period and the exercise period (i.e., the period of 5 to 10 seconds) defining the second half of the calibration period.

The start measurement button G31 is a button for starting the measurement processing. Tapping the start measurement button G31 causes the electroencephalogram measurement system 10 to start measuring an electroencephalogram.

The next button G32 is a button for making a transition to the calculation processing. Tapping the next button G32 causes the electroencephalogram measurement system 10 to end the measurement processing, thus changing the screens displayed on the display unit 26 of the information processor 2 from the calibration screen 202 to the selection screen 203 (see FIG. 8).

The back button G33 is a button for having the electroencephalogram measurement system 10 go back to the state before the calibration processing is started. Tapping the back button G33 causes the electroencephalogram measurement system 10 to end the calibration processing and go back to the state before the calibration processing is started.

The selection screen 203 includes a record display area G41, an operation guidance area G42, a legend display area G43, a retry addition button G44, a selected records number display area G45, and a calculate button G46 as shown in FIG. 8.

The record display area G41 is an area for displaying the electroencephalograms that are stored as records in the memory 22. In this case, in the electroencephalogram graphs displayed in the record display area G41, as well as in the electroencephalogram display area G2, waveforms representing variations in electroencephalogram with time are displayed with the abscissa indicating the time (in seconds) and the ordinate indicating the potential. In the example illustrated in FIG. 8, with respect to a plurality of (e.g., two in this example) records stored in the memory 22, electroencephalograms are displayed one on top of another. In addition, in the record display area G41, checkboxes G411 are also displayed on the right of the respective electroencephalograms in association with the respective records. The checkboxes G411 are icons for selecting a record for use in the calculation processing. Tapping any of these checkboxes G411 alternately switches the state where its associated record is used for the calculation processing to the state where the associated record is not used for the calculation processing, and vice versa. When any of these checkboxes G411 is active on the screen, its associated record is used for the calculation processing.

In this case, if there are too many records to be displayed as a list in the record display area G41, then the records to be displayed in the record display area G41 may be scrolled up and down. This allows a lot of records, of which the number is even greater than that of the records displayable as a list in the record display area G41, to be displayed in the record display area G41.

In addition, under each electroencephalogram displayed as a record in the record display area G41, an artifact mark M2 in a band shape is displayed in association with each record. The artifact mark M2 is shown only during a period in which an artifact is determined to be included in the electroencephalogram information. As described above, the electroencephalogram measurement system 10 according to this embodiment has the function (i.e., the decision unit 214) of determining whether or not any artifact is included in the electroencephalogram information. Thus, the decision made by the decision unit 214 will be reflected on the artifact mark M2.

That is to say, in the period in which the artifact mark M2 is displayed for a certain piece of electroencephalogram information, some artifact is highly likely included in that piece of electroencephalogram information. On the other hand, in the period in which the artifact mark M2 is not displayed for another piece of electroencephalogram information, no artifacts are highly likely included in that piece of electroencephalogram information. The electroencephalogram measurement system 10 according to this embodiment displays such artifact marks M2 in the record display area G41, thus allowing the subject 5 or the medical staff to easily pick a record for use in the calculation processing from among records with no artifacts. That is to say, having the subject 5 or the medical staff pick only records with no artifact marks M2 displayed when selecting records for use in the calculation processing using the checkboxes G411 allows only records with no artifacts to be extracted. This allows parameters to be determined accurately based on electroencephalogram information with no artifacts.

At this time, the input unit 216 accepts, from the operating unit 24, a designation signal indicating a record selected by either the subject 5 or the medical staff by checking off any of the checkboxes G411. That is to say, when either the subject 5 or the medical staff selects a record for use in the calculation processing by checking off any of the checkboxes G411, one or more sets of electroencephalogram information for use in the calibration processing (calculation processing) is designated from among multiple sets of electroencephalogram information acquired by the acquisition unit 211.

The operation guidance area G42 is an area for displaying text information providing some guidance to operate the electroencephalogram measurement system 10 properly. In the example illustrated in FIG. 8, displayed is text information saying "select waveform(s) for use in analysis. Select two or more waveforms and press "calculate" to display calculation results here. You may retry for unchecked waveform. If you want to retry, then press "retry addition" button." The message displayed in the operation guidance area G42 varies according to the operating condition of the electroencephalogram measurement system 10.

The legend display area G43 is an area for providing brief description of the display modes of the artifact marks M2. As will be described in detail later, the electroencephalogram measurement system 10 according to this embodiment has not only the function of determining whether or not there are any artifacts but also the function of determining the types of the artifacts if there should be any. The display mode of the artifact mark M2 varies according to the type of the artifact detected. In general, there are at least two types of artifacts, namely, an artifact deriving from an eye movement (hereinafter referred to as an "eye movement derived artifact") and an artifact deriving from a body movement or facial muscle movement (hereinafter referred to as a "body movement derived artifact"). The eye movement derived artifact and the body movement derived artifact have artifact marks M2 displayed in different modes (e.g., in two different colors). Therefore, in the legend display area G43, these two types of display modes are shown along with their brief description.

The retry addition button G44 is a button for starting an addition retry. As used herein, the "addition retry" refers to the processing of adding a record by performing the measurement processing all over again. Tapping the retry addition button G44 causes a transition to be made from the selection screen 203 to the calibration screen 202.

The selected records number display area G45 is an area for displaying the number of records (or waveforms) selected on the selection screen 203. Specifically, the number of records is shown as a fraction, which is the ratio of the number of records selected for use in the calculation processing (as the numerator) to the number of records displayed on the record display area G41 (i.e., the number of records stored in the memory 22) (as the denominator). For example, if the two records displayed in the record display area G41 are both selected (i.e., if the checkboxes G411 are both checked off) as shown in FIG. 8, then "2/2" is shown in the selected records number display area G45.

The calculate button G46 is a button for starting the calculation processing. Tapping the calculate button G46 causes the electroencephalogram measurement system 10 to start the calculation processing using the records selected at that time on the selection screen 203.

The calibration result screen 204, as well as the selection screen 203, includes the record display area G41, the legend display area G43, the retry addition button G44, the selected records number display area G45, and the calculate button G46 as shown in FIG. 9. In addition, the calibration result screen 204 further includes a time-frequency map G51, an information display area G52, an analysis result display area G53, and an end button G54.

The time-frequency map G51 is a graph showing the trend of a power variation in a particular frequency band during the calibration period used in the calculation processing. The time-frequency map G51 is a two-dimensional map, of which the abscissa indicates the time and the ordinate indicates the frequency and in which the power at each coordinate position is expressed by "color." That is to say, the time-frequency map G51 visually displays the frequency band in which the power varies in a transition period from the rest period to the exercise period. In addition, in the time-frequency map G51, also displayed are lines G511 indicating the frequency band of the α wave calculated through the calculation processing and lines G512 indicating the frequency band of the β wave calculated through the calculation processing. Specifically, the lines G511 indicate the lower and upper limit values of the frequency band (or range) of the α wave, and the lines G512 indicate the lower and upper limit values of the frequency band (or range) of the β wave.

The information display area G52 is an area for displaying information about the measurement processing. In the example illustrated in FIG. 9, pieces of text information, indicating the number of times the measurement processing has been performed (i.e., the number of analysis attempts), the time length of the rest period (relaxed period), and the time length of the exercise period (imagining period), are displayed in the information display area G52.

The analysis result display area G53 is an area for displaying the results of analysis obtained through the calculation processing. In the example illustrated in FIG. 9, pieces of text information indicating the frequency band of the α wave (α wave frequency range), the frequency band of the β wave (β wave frequency range), and the ratio at which an electroencephalogram with the characteristic variation has been produced due to the event-related desynchronization (accuracy rate) are displayed in the analysis result display area G53.

The end button G54 is a button for ending the calibration processing. Tapping the end button G54 causes the electroencephalogram measurement system 10 to end the calibration processing.

### (3.3.3) Artifact decision

Next, artifact decision processing to be carried out at the time of the calibration processing will be described in detail.

In this embodiment, the artifact marks M2 are displayed so as to allow either the subject 5 or the medical staff to pick only records with no artifacts when selecting records for use in the calculation processing during the calibration processing. Thus, the electroencephalogram measurement system 10 according to this embodiment includes not only the acquisition unit 211 for acquiring the electroencephalogram information but also the decision unit 214 and the output unit 217 as well. The electroencephalogram measurement system 10 makes the decision unit 214 determine whether or not there are any artifacts in the electroencephalogram information, and also makes the output unit 217 output the decision made by the decision unit 214.

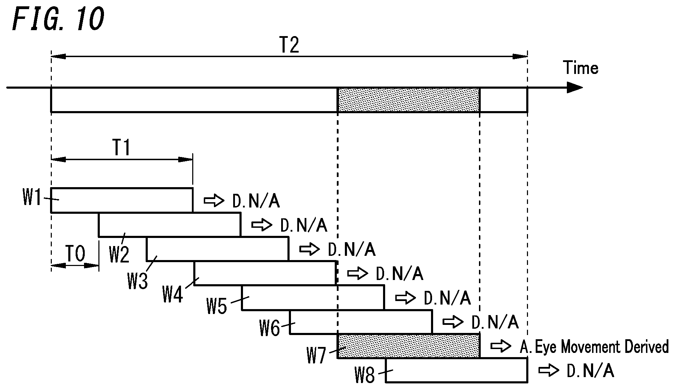

In this embodiment, supposing a second period T2 is allocated to each record, the decision unit 214 determines, for each of a plurality of (e.g., eight in this example) decision windows W1-W8 set within the second period T2, whether or not there are any artifacts as shown in FIG. 10. The plurality of decision windows W1-W8 each have the same time length and are arranged along the time axis so as to shift from each other by a predetermined amount of time T0. In this case, if the period of each of these decision windows W1-W8 is the first period T1, then the decision unit 214 determines, every first period T1, with respect to the second period T2 including a plurality of first periods T1 that are set along the time axis so as to shift from each other by the predetermined amount of time T0, whether or not there are any artifacts. The predetermined amount of time T0 is shorter than the time length of each of the first periods T1. Therefore, each of the plurality of decision windows W1-W8 has a period overlapping with the previous decision window. The decision unit 214 makes the artifact decision based on the ratio of the feature quantity of the electroencephalogram information during each of the plurality of first periods (i.e., the decision windows W1-W8) to the feature quantity of the electroencephalogram information during the second period T2.

As described above, each record is the time series data of electroencephalogram information measured by the electroencephalogram measurement system 10 since the beginning through the end of the calibration period (of 10 seconds). That is why a single record, i.e., the second period T2, has a time length of 10 seconds. Meanwhile, the predetermined amount of time T0 is supposed to be 1 second and the time length of the first period T1 is supposed to be 3 seconds.

As used herein, the "feature quantity" is the integrated value of the spectrum intensities of the electroencephalogram information on a predetermined frequency band, i.e., the "power." In short, according to this embodiment, the decision unit 214 makes the artifact decision based on the ratio of the power of the electroencephalogram during each of the plurality of first periods T1 (corresponding to the decision windows W1-W8) to the power of the electroencephalogram during the second period T2. Specifically, for each of the plurality of decision windows W1-W8, the decision unit 214 calculates the ratio of powers (hereinafter simply referred to as a "power ratio") as the ratio of the power in the predetermined frequency band in each of the first periods T1 (as the numerator) to the power in the predetermined frequency band in the second period T2 (as the denominator). Then, the decision unit 214 compares the power ratio calculated in this manner (i.e., the power in the predetermined frequency band in each first period T1/the power in the predetermined frequency band in the second period T2) with a prescribed value, thereby determining whether or not there is any artifact.

Furthermore, in the electroencephalogram measurement system 10 according to this embodiment, if the decision unit 214 has made a decision that there should be any artifact as described above, then the decision unit 214 further determines the type of the artifact. That is to say, if the decision unit 214 has determined that the electroencephalogram information should include any artifact, then the decision unit 214 also determines whether the type of the artifact is at least the eye movement derived artifact or the body movement derived artifact. FIG. 10 schematically illustrates an example in which the decision has been made that there should be an artifact in the decision window W7 and that the type of the artifact is determined to be the eye movement derived artifact.

Specifically, the decision unit 214 determines the type of the artifact based on either first electroencephalogram information or second electroencephalogram information and the difference between the first electroencephalogram information and the second electroencephalogram information. That is to say, in this embodiment, the electrode unit 11 includes the first electrode 111 and the second electrode 112, and therefore, the electroencephalogram information includes the first electroencephalogram information representing the electroencephalogram obtained by the first electrode 111 and the second electroencephalogram information representing the electroencephalogram obtained by the second electrode 112. Thus, first of all, the decision unit 214 calculates the power ratio with respect to either the first electroencephalogram information or the second electroencephalogram information (e.g., the first electroencephalogram information in this embodiment). Next, the decision unit 214 calculates the power ratio with respect to the difference between the first electroencephalogram information and the second electroencephalogram information.

More specifically, the power ratio with respect to the first electroencephalogram information is obtained over the entire frequency band covering both of the α wave range and the β wave range. Such a power ratio obtained with respect to the first electroencephalogram information over the entire frequency band covering both of the α wave range and the β wave range will be hereinafter referred to as an "entire frequency ratio R1." On the other hand, the power ratio with respect to the difference between the first electroencephalogram information and the second electroencephalogram information is obtained for only the frequency band of the β wave. Such a power ratio obtained for only the frequency band of the β wave with respect to the difference between the first electroencephalogram information and the second electroencephalogram information will be hereinafter referred to as a "β wave ratio R2."

In this case, generally speaking, a first period T1 with an artifact has a larger entire frequency ratio R1 than a first period T1 with no artifacts. Thus, the decision unit 214 determines, by comparing the entire frequency ratio R1 with the prescribed value V1, whether or not there is any artifact. Also, in general, in a first period T1 with the body movement derived artifact, the difference between the artifact included in the first electroencephalogram information and the artifact included in the second electroencephalogram information is more significant, and therefore, comes to have a larger β wave ratio R2, than in a first period T1 with the eye movement derived artifact. Thus, the decision unit 214 determines, by comparing the β wave ratio R2 with the prescribed value V2, whether the artifact derives from the body movement or the eye movement.

The output unit 217 has the capability of outputting the decision made by the decision unit 214. In this embodiment, the output unit 217 outputs the decision to the display control unit 218 such that at least the decision made by the decision unit 214 is displayed on the display unit 26. This allows the display control unit 218 to display, as the artifact mark M2, the decision made by the decision unit 214 on the selection screen 203 and the calibration result screen 204.

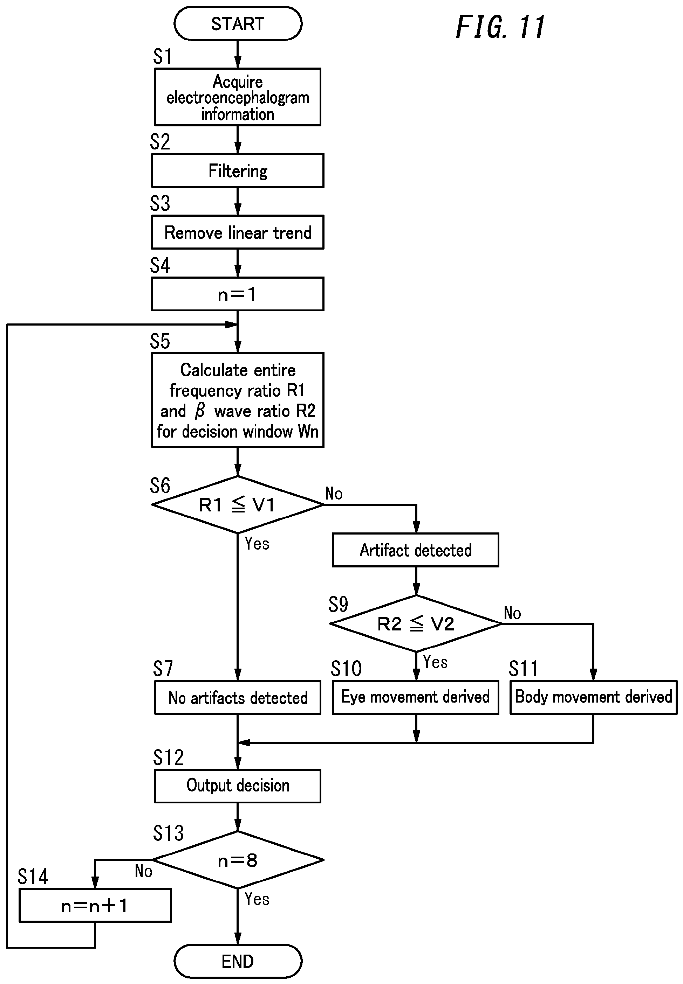

Next, it will be described with reference to the flowchart shown in FIG. 11 how to perform the artifact decision processing using the electroencephalogram measurement system 10 according to this embodiment.

First, the electroencephalogram measurement system 10 makes the acquisition unit 211 acquire electroencephalogram information (including first electroencephalogram information and second electroencephalogram information) (in Step S1). Next, the electroencephalogram measurement system 10 makes the decision unit 214 filter, through a bandpass filter and a notch filter, the electroencephalogram information thus acquired, thereby removing frequency bands unnecessary for analysis (in Step S2). Subsequently, the electroencephalogram measurement system 10 makes the decision unit 214 remove a linear trend (in Step S3).

Thereafter, the electroencephalogram measurement system 10 makes the decision unit 214 substitute "1" for a variable n (in Step S4), thereby performing the processing of calculating the entire frequency ratio R1 and the β wave ratio R2 with respect to the decision window Wn (in Step S5). Next, the electroencephalogram measurement system 10 makes the decision unit 214 compare the entire frequency ratio R1 with the prescribed value V1 (in Step S6). When finding the entire frequency ratio R1 equal to or less than the prescribed value V1 (if the answer is YES in Step S6), the decision unit 214 makes a decision that there should be no artifacts with respect to the decision window Wn (in Step S7).

On the other hand, when finding the entire frequency ratio R1 larger than the prescribed value V1 (if the answer is NO in Step S6), the decision unit 214 makes a decision that there should be some artifact with respect to the decision window Wn (in Step S8) and determines the type of the artifact (in Step S9). That is to say, the electroencephalogram measurement system 10 makes the decision unit 214 compare a β wave ratio R2 with a prescribed value V2 (in Step S9). In this case, when finding the β wave ratio R2 equal to or less than the prescribed value V2 (if the answer is YES in Step S9), the decision unit 214 makes a decision that the type of the artifact included in the decision window Wn should be an eye movement derived artifact (in Step S10). On the other hand, when finding the β wave ratio R2 greater than the prescribed value V2 (if the answer is NO in Step S9), the decision unit 214 makes a decision that the type of the artifact included in the decision window Wn should be a body movement derived artifact (in Step S11).

After having performed Step S7, S10, or S11, the electroencephalogram measurement system 10 makes the output unit 217 output the decision made by the decision unit 214 to the display control unit 218 (in Step S12). As a result, the decision made by the decision unit 214 is displayed on the display unit 26. Next, the electroencephalogram measurement system 10 makes the decision unit 214 determine whether or not the variable n has reached 8 (in Step S13). Unless the variable n has reached 8 yet (if the answer is NO in Step S13), the electroencephalogram measurement system 10 makes the decision unit 214 substitute "n+1" for the variable n (in Step S14) and the process goes back to the processing step S5. When finding the variable n has reached 8 (if the answer is YES in Step S13), the artifact decision processing ends.

### (Variations)

The first embodiment described above is only one of various embodiments of the present disclosure, and may be readily modified or changed depending on a design choice or any other factor, without departing from the scope of the present disclosure. Also, the same function as that of the electroencephalogram measurement system 10 may also be implemented as an electroencephalogram measurement method, a (computer) program, or a non-transitory storage medium that stores the computer program thereon, for example. An electroencephalogram measurement method according to an aspect includes: acquiring electroencephalogram information representing an electroencephalogram obtained by an electrode unit 11 placed on a region of interest 51 that forms part of the subject's 5 head 52; making a decision, based on the electroencephalogram information acquired, whether or not there are any artifacts; and outputting the decision made about the artifacts. A (computer) program according to another aspect is designed to cause a computer system to carry out the electroencephalogram measurement method described above.

Next, variations of the first embodiment described above will be enumerated one after another. Note that any of the variations to be described below may be combined as appropriate.

The electroencephalogram measurement system 10 according to the present disclosure includes a computer system. In that case, the computer system may include, as principal hardware components, a processor and a memory. The functions of the electroencephalogram measurement system 10 according to the present disclosure may be performed by making the processor execute a program stored in the memory of the computer system. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be made up of a single or a plurality of electronic circuits including a semiconductor integrated circuit (IC) or a largescale integrated circuit (LSI). As used herein, the "integrated circuit" such as an IC or an LSI is called by a different name depending on the degree of integration thereof. Examples of the integrated circuits include a system LSI, a very largescale integrated circuit (VLSI), and an ultra largescale integrated circuit (ULSI). Optionally, a field-programmable gate array (FPGA) to be programmed after an LSI has been fabricated or a reconfigurable logic device allowing the connections or circuit sections inside of an LSI to be reconfigured may also be adopted as the processor. Those electronic circuits may be either integrated together on a single chip or distributed on multiple chips, whichever is appropriate. Those multiple chips may be integrated together in a single device or distributed in multiple devices without limitation.

Also, in the embodiment described above, the plurality of constituent elements of the information processor 2 are integrated together in a single housing. However, this is not a configuration essential to the electroencephalogram measurement system 10. Alternatively, those constituent elements of the information processor 2 may be distributed in multiple different housings. Even when a plurality of constituent elements are distributed in multiple different housings, those constituent element are still able to serve as the electroencephalogram measurement system 10 in conjunction with each other when connected together via a network such as the Internet. Still alternatively, at least some functions of the electroencephalogram measurement system 10 may be implemented as a server or a cloud computing system as well. Conversely, the plurality of functions distributed in multiple devices such as the headset 1 and the information processor 2 may be integrated as parts of the electroencephalogram measurement system 10, along with the other constituent elements of the electroencephalogram measurement system 10, in a single housing.

Also, the electrode unit 11 does not have to be configured to come into contact with the surface of the subject's 5 head 52 (i.e., the scalp). Alternatively, the electrode unit 11 may also be configured to come into contact with the surface of the brain, for example.

Furthermore, the rehabilitation support system 100 does not always support the subject 5 in his or her rehabilitation to recover the proper function of his or her hand fingers but may also support the subject 5 in his or her rehabilitation for any other body region such as his or her shoulder(s), elbow(s), upper arm(s), waist, lower limbs, or upper limbs. The characteristic variation of an electroencephalogram that may arise when the subject 5 plans to do (or imagines doing) voluntary movement may vary depending on the target region of the rehabilitation or the type of the movement. For example, if an event-related synchronization (ERS) occurs when the subject 5 plans to do the voluntary movement, the power in a particular frequency band increases in the electroencephalogram representing a brain wave measured in the vicinity of the motor area during the voluntary movement. **In** that case, the electroencephalogram measurement system 10 detects, by seeing the power in the particular frequency band increase, the characteristic variation in electroencephalogram.

Furthermore, the rehabilitation support system 100 does not have to be configured to apply either an electrical stimulus or a mechanical (or dynamic) stimulus to the subject 5 but may also be configured to apply a visual stimulus to the subject 5 by presenting virtual video to him or her. **In** that case, when the subject 5 plans to do the voluntary movement, the rehabilitation support system 100 presents virtual video, representing his or her affected region as if the affected region were functioning normally, to him or her at the timing when his or her brain region corresponding to the target region of the voluntary movement is actually activated. This also allows the rehabilitation support system 100 to support the subject 5 in his or her voluntary movement.

Furthermore, the exercise assisting device 3 and the controller 4 do not have to be provided separately from each other. Alternatively, the exercise assisting device 3 and the controller 4 may also be housed and integrated together in the same housing.

Furthermore, the method of communication between the headset 1 and the information processor 2 is supposed to be wireless communication in the first embodiment described above, but may also be wired communication or communications via a relay, for example. Furthermore, the method of communication between the controller 4 and the information processor 2 is supposed to be wired communication in the first embodiment but may also be wireless communication or communication via a relay, for example.

Furthermore, the headset 1 does not have to be driven by a battery but the power to operate the signal processing unit 12, the first communications unit 13, and other components may also be supplied from the information processor 2, for example.

Furthermore, the information processor 2 does not have to be configured to acquire electroencephalogram information from the dedicated headset 1. Alternatively, the information processor 2 may also be configured to acquire electroencephalogram information from a general-purpose electroencephalograph, for example.

Furthermore, in the first embodiment described above, the result of the artifact decision is used for the calibration processing. However, this is only an example and should not be construed as limiting. Alternatively, the result of the artifact decision may also be output during the training process, for example. This allows either the subject 5 or the medical staff to easily confirm the condition of the artifact during the training process.

Furthermore, in the first embodiment described above, either the subject 5 or the medical staff selects electroencephalogram information for use in the calibration processing by reference to the result of the artifact decision. However, this is only an example and should not be construed as limiting. Alternatively, the electroencephalogram information for use in the calibration processing may also be selected automatically. Specifically, in that case, the output unit 217 may output the result of the artifact decision to the processing unit 215, for example, to make the processing unit 215 automatically select, based on the result of the artifact decision, the electroencephalogram information for use in the calibration processing. In that case, the processing unit 215 suitably selects only the electroencephalogram information that has turned out to have no artifacts.

Furthermore, if one of two values (such as the entire frequency ratio R1 and the prescribed value V1) being compared with each other is "equal to or less than" the other, the phrase "equal to or less than" covers both a situation where these two values are equal to each other and a situation where one of the two values is less than the other. However, this is only an example and should not be construed as limiting. The phrase "equal to or less than" may also be a synonym of the phrase "less than" that covers only a situation where one of the two values is less than the other. That is to say, it is arbitrarily changeable, depending on selection of a threshold value or any preset value, whether or not the phrase "less than" covers the situation where the two values are equal to each other. Therefore, from a technical point of view, there is no difference between the phrase "less than" and the phrase "equal to or less than." Similarly, the phrase "greater than" may be a synonym of the phrase "equal to or greater than."

### (Second embodiment)

An electroencephalogram measurement system 10 according to a second embodiment uses a different artifact decision method from the electroencephalogram measurement system 10 according to the first embodiment described above. In the following description, any constituent element of this second embodiment, having the same function as a counterpart of the first embodiment described above, will be designated by the same reference numeral as that counterpart's, and description thereof will be omitted herein as appropriate.

First of all, according to this embodiment, the algorithm of the artifact decision processing to be performed at the time of the calibration processing may be selected from a plurality of algorithms. In other words, the algorithm for use in the artifact decision processing may be designated from among the plurality of algorithms. The algorithm may be selected, for example, by either the subject 5 or the medical staff on the screen (which may be the calibration screen 202, the selection screen 203, or the calibration result screen 204) displayed on the display unit 26 of the information processor 2 at the time of the calibration processing. That is to say, the input unit 216 accepts, from the operating unit 24, a designation signal representing the algorithm selected by either the subject 5 or the medical staff. Alternatively, the algorithm may be selected automatically depending on the type of file loading at the acquisition unit 211.

The plurality of algorithms includes at least a first algorithm and a second algorithm. The first algorithm is a simple algorithm, while the second algorithm is a complicated algorithm. The first algorithm is an algorithm for making the artifact decision by arithmetic processing which is simple enough for a human (such as the subject 5 or the medical staff) to understand and may be implemented as a decision tree, for example. On the other hand, the second algorithm is an algorithm for making the artifact decision by arithmetic processing which is too complicated for a human (such as the subject 5 or the medical staff) to understand and may be implemented as deep learning, for example.

Comparing the first algorithm with the second algorithm, it can be seen that the second algorithm has higher decision accuracy than the first algorithm, the second algorithm also has higher time resolution than the first algorithm, the second algorithm is able to make a decision about a wider variety of artifacts than the first algorithm, and the second algorithm requires a longer time to get computations done than the first algorithm. That is why selecting the second algorithm improves the decision accuracy so much as to make even minor artifacts, which would be missed by the first algorithm, detectable. In addition, selecting the second algorithm increases the time resolution and the number of artifacts to make decision about so much as to allow the medical staff to give the subject 5 more pointed instructions for reducing the artifacts at an even better timing. On the other hand, selecting the first algorithm reduces the chances of arousing doubt as to the decision that there should be some artifacts because the first algorithm is understandable for a human (such as the subject 5 or the medical staff). In addition, selecting the first algorithm shortens the waiting time for the subject 5 or the medical staff because the first algorithm requires a shorter time to get computations done than the second algorithm does.

Making the algorithm for the artifact decision processing selectable from among a plurality of algorithms including the first algorithm and the second algorithm in this manner achieves the following advantages. Specifically, it depends on the knowledge or skills of each individual subject 5 or medical staff which algorithm is preferably selected for him or her, the first algorithm or second algorithm. For example, if the subject 5 or the medical staff is knowledgeable about electroencephalograms, the simpler, first algorithm would be more preferable for him or her. This is because the subject 5 or medical staff knowledgeable about electroencephalograms prefers understandable artifact decision results and is also able to complement even inaccurate decision results with his or her own knowledge. On the other hand, if the subject 5 or the medical staff is not knowledgeable about electroencephalograms, then the more complicated, second algorithm would be more preferable for him or her. This is because the subject 5 or medical staff who is not knowledgeable about electroencephalograms prefers to leave the artifact decision to the electroencephalogram measurement system 10.

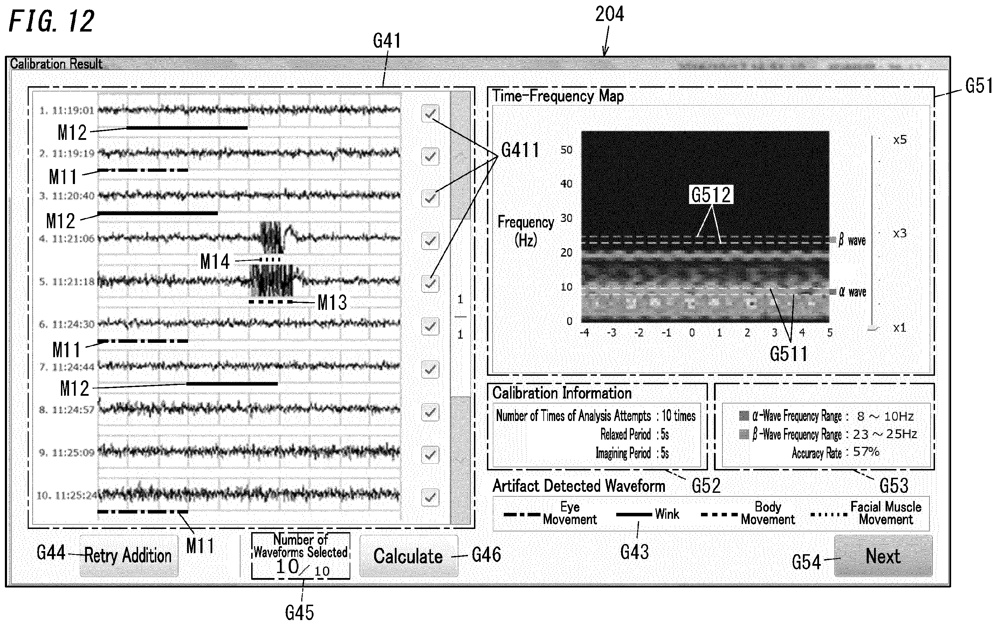

FIG. 12 shows an exemplary calibration result screen 204 in a situation where the artifact decision processing has been performed using the second algorithm. On the other hand, performing the artifact decision processing on the same record (waveform) as the one shown in FIG. 12 using the first algorithm would produce the result (i.e., calibration result screen 204) shown in FIG. 9.

Specifically, using the second algorithm causes band-shaped artifact marks M11-M14 to be displayed under the respective records (electroencephalograms) in the record display area G41 as shown in FIG. 12. Each of these artifact marks M11-M14 is shown only during the period in which the decision is made that some artifact should be included in the electroencephalogram information.

Unlike the artifact marks M2 (see FIG. 9) described for the first embodiment, these artifact marks M11-M14 are displayed on an individual basis for the four types of artifacts, respectively. That is to say, the second algorithm is able to make a decision about a wider variety of artifacts than the first algorithm does, and therefore, the electroencephalogram measurement system 10 makes a decision about three or more types (e.g., four types in this embodiment) of artifacts and distinguishes those artifacts from each other. In this case, the four types of artifacts derive from an eye movement, a wink, a body movement, and a facial muscle movement, respectively, as described in the legend display area G43. Thus, compared to the first algorithm that makes a decision about only two types of artifacts, namely, the eye movement derived artifact and the body movement derived artifact, with these two types distinguished from each other, the second algorithm facilitates determination of the cause of an artifact based on the decision result. This allows the medical staff to easily determine the subject's 5 movement that has caused the artifact and give the subject 5 more pointed instructions for reducing the artifacts. For example, if an artifact deriving from a wink has been caused, then the medical staff may give the subject 5 instructions to refrain from winking.

In addition, as can also be seen from FIG. 12, the artifact marks M11-M14 are activated less redundantly than the artifact marks M2 (see FIG. 9) described for the first embodiment with respect to the periods during which the artifacts are actually caused. That is to say, the second algorithm has higher time resolution than the first algorithm, and therefore, allows the electroencephalogram measurement system 10 to make artifact decisions for virtually only the periods during which the artifacts are actually caused. This allows the medical staff to give the subject 5 instructions for reducing the artifacts at a more appropriate timing.

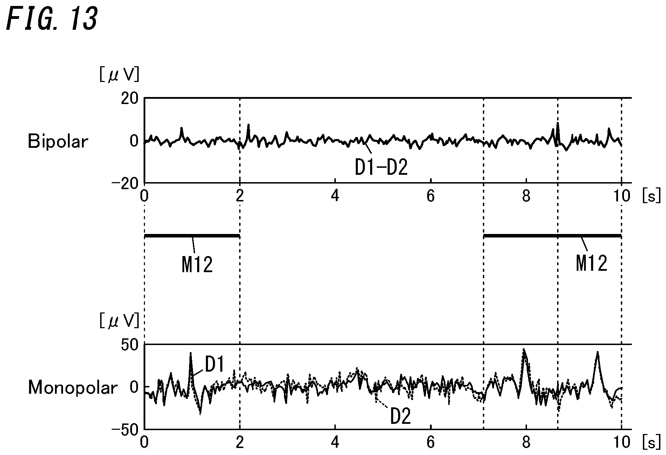

FIG. 13 is a waveform diagram, of which the abscissa indicates the time (in seconds) and the ordinate indicates the potential and which represents variations in electroencephalogram with time with respect to each of the electroencephalogram derived by bipolar derivation and the electroencephalogram derived by monopolar derivation. In FIG. 13, the upper graph labeled "Bipolar" represents the electroencephalogram derived by bipolar derivation, and the lower graph labeled "Monopolar" represents the electroencephalogram derived by monopolar derivation. In this case, the electroencephalogram derived by monopolar derivation includes first electroencephalogram information D1 representing an electroencephalogram obtained by the first electrode 111 and second electroencephalogram information D2 representing an electroencephalogram obtained by the second electrode 112. The first electroencephalogram information D1 and the second electroencephalogram information D2 represent the potentials of the first electrode 111 and the second electrode 112, respectively, with respect to the potential of the reference electrode 113 as a reference potential. On the other hand, the electroencephalogram derived by bipolar derivation is the difference (D1 - D2) between the first electroencephalogram information D1 and the second electroencephalogram information D2. In FIG. 13, an artifact is supposed to have been caused due to a wink (as indicated by artifact marks M2).

In this embodiment, no matter whether the first algorithm or the second algorithm is adopted, both the electroencephalogram (including the first electroencephalogram information D1 and the second electroencephalogram information D2) derived by monopolar derivation and the electroencephalogram (D1 - D2) derived by bipolar derivation are used to make artifact decisions. In general, an artifact derived from an eye movement similarly affects the first electrode 111 and second electrode 112, which are arranged close to each other. Thus, when an artifact derived from an eye movement is caused, the first electroencephalogram information D1 and the second electroencephalogram information D2 vary in similar patterns. Therefore, as can be seen from FIG. 13, when an artifact is caused due to a wink, a variation due to the eye movement derived artifact is less likely to be caused in the electroencephalogram (D1 - D2) derived by bipolar derivation. Thus, as shown in FIG. 13, the electroencephalogram (D1 - D2) derived by bipolar derivation seems as if no artifacts had been caused. For these reasons, a decision about this type of artifact is made using the electroencephalogram (including the first electroencephalogram information D1 and the second electroencephalogram information D2) derived by monopolar derivation.

In this embodiment, the electroencephalogram (D1 - D2) derived by bipolar derivation is displayed on the calibration result screen 204 shown in FIG. 12, for example. Therefore, on the calibration result screen 204, an electroencephalogram showing no signs of variation caused by an artifact and the result of the artifact decision (i.e., the artifact mark M12) are displayed in combination. That is to say, the display control unit 218 makes the display unit 26 display, along with the decision result (i.e., the artifact marks M12), the waveform of the electroencephalogram derived by a derivation method (i.e., the bipolar derivation in this case) different from a derivation method (i.e., the monopolar derivation in this case) for deriving the electroencephalogram waveform used to make the decision.

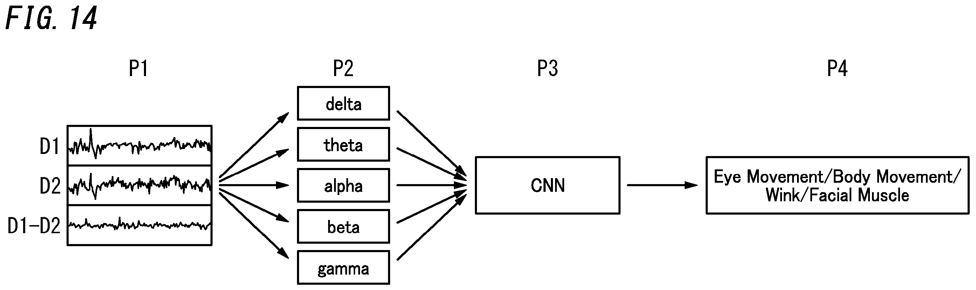

Next, exemplary artifact decision processing using, as the second algorithm, a convolutional neural network (CNN) will be described with reference to FIG. 14. FIG. 14 is a conceptual diagram schematically illustrating the flow of such artifact decision processing.

Specifically, in this example, the decision processing includes a first processing step P1, a second processing step P2, a third processing step P3, and a fourth processing step P4, when classified roughly. First, in the first processing step P1, data of respective decision windows W1-W8 (see FIG. 9) is extracted from the records of the electroencephalogram (including the first electroencephalogram information D1 and the second electroencephalogram information D2) derived by monopolar derivation and the electroencephalogram (D1 - D2) derived by bipolar derivation. Next, in the second processing step P2, the power ratio is calculated on a frequency band (α, β, γ, δ, ζ) basis with respect to each of the electroencephalogram (including the first electroencephalogram information D1 and the second electroencephalogram information D2) and the electroencephalogram (D1 - D2) derived by bipolar derivation. Subsequently, in the third processing step P3, the power ratios thus extracted on a frequency band basis are input to the convolutional neural network (designated by CNN in FIG. 14). Thereafter, in the fourth processing step P4, information indicating whether any artifact is present or not and information about the type of the artifact (i.e., an artifact deriving from an eye movement, a body movement, a wink, or a facial muscle movement) if any are output from the convolutional neural network. The artifact decision processing by the second algorithm is carried out by performing this series of processing steps.

Note that the algorithms described above are only examples. That is to say, the first algorithm does not have to be a decision tree but may also be implemented by a linear classifier, a random forest, or a nearest neighbor algorithm, for example. The second algorithm does not have to be implemented as the convolutional neural network but may also be implemented as a recurrent neural network or a support vector machine, for example.

Note that the various configurations (including variations) described for the second embodiment may be adopted, as appropriate, in combination with the various configurations (including variations) described for the first embodiment.

### Reference Signs List

- 5: Subject
- 10: Electroencephalogram Measurement System
- 11: Electrode Unit
- 24: Operating Unit
- 26: Display Unit
- 51: Region of Interest
- 52: Head
- 111: First Electrode
- 112: Second Electrode
- 211: Acquisition Unit
- 214: Decision Unit
- 215: Processing Unit
- 216: Input Unit
- 217: Output Unit
- 218: Display Control Unit
- 511: First Region of Interest
- 512: Second Region of Interest
- T1: First Period
- T2: Second Period

## Claims

1. An electroencephalogram measurement system (10) comprising:
an acquisition unit (211) configured to acquire electroencephalogram information, the electroencephalogram information representing an electroencephalogram obtained by an electrode unit (11), the electrode unit (11) being placed on a region of interest (51) that forms part of a subject's head (52);
a decision unit (214) configured to make a decision, based on the electroencephalogram information acquired by the acquisition unit (211), whether or not there are any artifacts;
an output unit (217) configured to output the decision made by the decision unit (214);
an input unit (216) configured to accept a designation signal designating one or more sets of the electroencephalogram information, among multiple sets of the electroencephalogram information acquired by the acquisition unit (211), the designation signal being input through an operating unit (24); and
a processing unit (215) configured to perform, based on the one or more sets of the electroencephalogram information designated by the designation signal, calibration processing for determining a parameter for use to analyze the electroencephalogram information,
wherein the electroencephalogram measurement system (10) is configured such that a selection screen (203) is displayed on a display unit (26),
wherein the selection screen (203) includes a record display area (G41), the record display area (G41) being an area for displaying the electroencephalograms,
wherein the electroencephalogram measurement system (10) is configured such that, under each electroencephalogram displayed as a record in the record display area (G41), an artifact mark (M2) in a band shape is displayed in association with each record,
wherein the artifact mark (M2) is shown during a period in which an artifact is determined to be included in the electroencephalogram information, such that the decision made by the decision unit (214) is reflected on the artifact mark (M2),
wherein,
when the decision made by the decision unit (214) is that there is an artifact, the decision unit (214) is configured to further determine whether a type of the artifact is an eye movement derived artifact or a body movement derived artifact,
wherein
the region of interest (51) includes a first region of interest (511) and a second region of interest (512),
the electrode unit (11) includes a first electrode (111) corresponding to the first region of interest (511) and a second electrode (112) corresponding to the second region of interest (512),
the acquisition unit (211) is configured to acquire first electroencephalogram information representing an electroencephalogram obtained by the first electrode (111) and second electroencephalogram information representing an electroencephalogram obtained by the second electrode (112), and
the decision unit (214) is configured to determine, based on either the first electroencephalogram information or the second electroencephalogram information and a difference between the first electroencephalogram information and the second electroencephalogram information, the types of the artifacts,
wherein
the decision unit (214) is configured to make the decision about the artifacts based on a ratio of a feature quantity of the electroencephalogram information during each of a plurality of first periods (T1) to a feature quantity of the electroencephalogram information during a second period (T2), the second period (T2) including the plurality of first periods (T1) that are set so as to shift from each other by a predetermined amount of time along a time axis,
wherein
the feature quantity is an integrated value of spectrum intensities of the electroencephalogram information on a predetermined frequency band.

2. The electroencephalogram measurement system (10) of claim 1, further comprising a display control unit (218) configured to make a display unit (26) display the decision output by the output unit (217).

3. The electroencephalogram measurement system (10) of claim 2, wherein
the display control unit (218) is configured to make the display unit (26) display, along with the decision, an electroencephalogram waveform derived by a derivation method different from a derivation method for deriving an electroencephalogram waveform used to make the decision.

4. An electroencephalogram measurement method comprising:
acquiring electroencephalogram information representing an electroencephalogram obtained by an electrode unit (11), the electrode unit (11) being placed on a region of interest (51) that forms part of a subject's head (52);
making a decision, based on the electroencephalogram information acquired, whether or not there are any artifacts;
outputting the decision made about the artifacts;
accepting a designation signal designating one or more sets of the electroencephalogram information, among multiple sets of the electroencephalogram information, the designation signal being input through an operating unit (24);
performing, based on the one or more sets of the electroencephalogram information designated by the designation signal, calibration processing for determining a parameter for use to analyze the electroencephalogram information;
displaying a selection screen (203) on a display unit (26),
wherein the selection screen (203) includes a record display area (G41), the record display area (G41) being an area for displaying the electroencephalograms,
wherein, under each electroencephalogram displayed as a record in the record display area (G41), an artifact mark (M2) in a band shape is displayed in association with each record, wherein the artifact mark M2 is shown during a period in which an artifact is determined to be included in the electroencephalogram information, such that the decision made by the decision unit (214) is reflected on the artifact mark (M2),
when the decision is that there is an artifact, further determining whether a type of the artifact is an eye movement derived artifact or a body movement derived artifact,
wherein
the region of interest (51) includes a first region of interest (511) and a second region of interest (512),
the electrode unit (11) includes a first electrode (111) corresponding to the first region of interest (511) and a second electrode (112) corresponding to the second region of interest (512), wherein the method further includes
acquiring first electroencephalogram information representing an electroencephalogram obtained by the first electrode (111) and second electroencephalogram information representing an electroencephalogram obtained by the second electrode (112),
determining, based on either the first electroencephalogram information or the second electroencephalogram information and a difference between the first electroencephalogram information and the second electroencephalogram information, the types of the artifacts, and
making the decision about the artifacts based on a ratio of a feature quantity of the electroencephalogram information during each of a plurality of first periods (T1) to a feature quantity of the electroencephalogram information during a second period (T2), the second period (T2) including the plurality of first periods (T1) that are set so as to shift from each other by a predetermined amount of time along a time axis,
wherein the feature quantity is an integrated value of spectrum intensities of the electroencephalogram information on a predetermined frequency band.

5. A computer program comprising instructions to cause the electroencephalogram measurement system according to claim 1 to carry out the electroencephalogram measurement method of claim 4.

6. A non-transitory computer-readable storage medium storing the program of claim 5.

## Patentansprüche

1. Elektroenzephalogrammmesssystem (10) umfassend:
eine Erfassungseinheit (211), die konfiguriert ist, Elektroenzephalogrammminformationen zu erfassen, wobei die Elektroenzephalogrammminformationen ein Elektroenzephalogrammm darstellen, das durch eine Elektrodeneinheit (11) erhalten wird, wobei die Elektrodeneinheit (11) auf eine Region von Interesse (51) positioniert wird, die Teil des Kopfes (52) eines Subjekts bildet;
eine Entscheidungseinheit (214), die konfiguriert ist, eine Entscheidung auf der Basis der Elektroenzephalogrammminformationen zu treffen, die durch die Erfassungseinheit (211) erfasst worden sind, ob irgendwelche Artefakte vorliegen oder nicht;
eine Ausgabeeinheit (217), die konfiguriert ist, die durch die Entscheidungseinheit (214) getroffene Entscheidung auszugeben;
eine Eingabeeinheit (216), die konfiguriert ist, ein Kennzeichnungssignal anzunehmen, das einen oder mehrere Sätze von Elektroenzephalogrammminformationen unter multiplen Sätzen der Elektroenzephalogrammminformationen kennzeichnet, die durch die Erfassungseinheit (211) erfasst worden ist, wobei das Kennzeichnungssignal durch eine Bedienungseinheit (24) eingegeben wird, und
eine Bearbeitungseinheit (215), die konfiguriert ist, auf der Basis eines oder mehrerer Sätze der Elektroenzephalogrammminformationen, die durch das Kennzeichnungssignal gekennzeichnet sind, Kalibrierungsbearbeiten zum Bestimmen eines Parameters für die Verwendung zum Analysieren der Elektroenzephalogrammminformationen durchzuführen,
wobei das Elektroenzephalogrammmesssystem (10) so konfiguriert ist, dass ein Wählbildschirm (203) auf einer Anzeigeeinheit (26) angezeigt wird,
wobei der Wählbildschirm (203) einen Aufnahmeanzeigebereich (G41) umfasst, wobei der Aufnahmeanzeigebereich (G41) ein Bereich zum Anzeigen der Elektroenzephalogrammme ist,
wobei das Elektroenzephalogrammmesssystem (10) so konfiguriert ist, dass unter jedem Elektroenzephalogrammm, das als Aufnahme im Aufnahmeanzeigebereich (G41) angezeigt ist, eine Artefaktmarkierung (M2)in einer Bandgestalt in Verbindung mit jeder Aufnahme angezeigt ist,
wobei die Artefaktmarkierung (M2) während einer Zeitspanne gezeigt wird, während der ein Artefakt dahingehend bestimmt wird, in den Elektroenzephalogrammminformationen enthalten zu sein, derart, dass die Entscheidung, die durch die Entscheidungseinheit (214) getroffen wird, auf der Artefaktmarkierung (M2) reflektiert wird,
wenn die Entscheidung, die durch die Entscheidungseinheit (214) getroffen wird, dahingehend ist, dass ein Artefakt vorliegt, die Entscheidungseinheit (214) konfiguriert wird, noch weiter zu bestimmen, ob ein Typ des Artefakts ein von einer Augenbewegung stammendes Artefakt oder ein von einer Körperbewegung stammendes Artefakt ist,
wobei
die Region von Interesse (51) eine erste Region von Interesse (511) und eine zweite Region von Interesse (512) umfasst,
die Elektrodeneinheit (11) eine erst Elektrode (111), die der ersten Region von Interesse (511) entspricht, und eine zweite Elektrode (112), die der zweiten Region von Interesse (512) entspricht, umfasst,
die Erfassungseinheit (211) konfiguriert ist, die ersten Elektroenzephalogrammminformationen, die ein Elektroenzephalogrammm darstellen, das durch die erste Elektrode (111) erhalten wird, und zweite Elektroenzephalogrammminformationen, die ein Elektroenzephalogrammm darstellen, das durch die zweite Elektrode (112) erhalten wird, zu erfassen und
die Entscheidungseinheit (214) konfiguriert ist, auf der Basis von entweder den ersten Elektroenzephalogrammminformationen oder den zweiten Elektroenzephalogrammminformationen und einem Unterschied zwischen den ersten Elektroenzephalogrammminformationen und den zweiten Elektroenzephalogrammminformationen die Typen der Artefakte zu bestimmen,
wobei
die Entscheidungseinheit (214) konfiguriert ist, die Entscheidung über die Störmeldungen auf der Basis eines Verhältnisses einer Merkmalmenge der Elektroenzephalogrammminformationen während jeder von einer Mehrzahl von ersten Zeitspannen (T1) zu einer Merkmalmenge der Elektroenzephalogrammminformationen während einer zweiten Zeitspanne (T2) zu treffen, wobei die zweite Zeitspanne (T2) die Mehrzahl erster Zeitspannen (T1) umfasst, die so festgelegt sind, sich voneinander durch eine vorbestimmte Zeitmenge entlang einer Zeitachse zu verschieben,
wobei
die Merkmalmenge ein integrierter Wert von Spektrumintensitäten der Elektroenzephalogrammminformationen auf einem vorbestimmten Frequenzband ist.

2. Elektroenzephalogrammmesssystem (10) nach Anspruch 1, ferner eine Anzeigekontrolleinheit (218) umfassend, die konfiguriert ist, eine Anzeigeeinheit (26) dazu zu bringen, die Entscheidungsausgabe durch die Ausgabeeinheit (217) anzuzeigen.

3. Elektroenzephalogrammmesssystem (10) nach Anspruch 1 2, wobei die Anzeigekontrolleinheit (218) konfiguriert ist, die Anzeigeeinheit (26) dazu zu bringen, zusammen mit der Entscheidung eine Elektroenzephalogrammm-Wellenform anzuzeigen, die durch ein Ableitungsverfahren abgeleitet ist, das von einem Ableitungsverfahren zum Ableiten einer Elektroenzephalogrammm-Wellenform, die zum Treffen der Entscheidung verwendet wird, verschieden ist.

4. Elektroenzephalogrammmesssystem (10) umfassend:
Erfassen von Elektroenzephalogrammminformationen, die ein Elektroenzephalogramm darstellen, das durch eine Elektrodeneinheit (11) erhalten wird, wobei die Elektrodeneinheit (11) auf eine Region von Interesse (51) positioniert wird, die Teil des Kopfes (52) eines Subjekts bildet;
Treffen einer Entscheidung auf der Basis der erfassten Elektroenzephalogrammminformationen, ob irgendwelche Artefakte vorliegen oder nicht;
Ausgeben der Entscheidung, die über die Artefakte getroffen worden ist;
Annehmen eines Kennzeichnungssignals, das einen oder mehrere Sätze von Elektroenzephalogrammminformationen unter multiplen Sätzen von Elektroenzephalogrammminformationen kennzeichnet, wobei das Kennzeichnungssignal durch eine Bedienungseinheit (24) eingegeben wird,
Ausführen, auf der Basis eines oder mehrerer Sätze der Elektroenzephalogrammminformationen, die durch das Kennzeichnungssignal gekennzeichnet sind, von Kalibrierungsbearbeiten zum Bestimmen eines Parameters für die Verwendung zum Analysieren der Elektroenzephalogrammminformationen,
Anzeigen eines Wählbildschirms (203) auf einer Anzeigeeinheit (26),
wobei der Wählbildschirm (203) einen Aufnahmeanzeigebereich (G41) umfasst, wobei der Aufnahmeanzeigebereich (G41) ein Bereich zum Anzeigen der Elektroenzephalogrammme ist,
wobei unter jedem Elektroenzephalogrammm, das als Aufnahme im Aufnahmeanzeigebereich (G41) angezeigt ist, eine Artefaktmarkierung (M2) in einer Bandgestalt in Verbindung mit jeder Aufnahme angezeigt ist, wobei die Artefaktmarkierung (M2) während einer Zeitspanne gezeigt wird, während der ein Artefakt dahingehend bestimmt wird, in den Elektroenzephalogrammminformationen enthalten zu sein, derart, dass die Entscheidung, die durch die Entscheidungseinheit (214) getroffen wird, auf der Artefaktmarkierung (M2) reflektiert wird,
wenn die Entscheidung dahingehend ist, dass ein Artefakt vorliegt, noch weiter bestimmen, ob ein Typ des Artefakts ein von einer Augenbewegung stammendes Artefakt oder ein von einer Körperbewegung stammendes Artefakt ist,
wobei
die Region von Interesse (51) eine erste Region von Interesse (511) und eine zweite Region von Interesse von Interesse (512) umfasst,
die Elektrodeneinheit (11) eine erst Elektrode (111), die der ersten Region von Interesse (511) entspricht, und eine zweite Elektrode (112), die der zweiten Region von Interesse (512) entspricht, umfasst,
wobei das Verfahren ferner Folgendes umfasst
Erfassen erster Elektroenzephalogrammminformationen, die ein Elektroenzephalogrammm darstellen, das durch die erste Elektrode (111) erhalten wird, und zweiter Elektroenzephalogrammminformationen, die ein Elektroenzephalogrammm darstellen, das durch die zweite Elektrode (112) erhalten wird,
Bestimmen, auf der Basis entweder der ersten Elektroenzephalogrammminformationen oder der zweiten Elektroenzephalogrammminformationen und eines Unterschieds zwischen den ersten Elektroenzephalogrammminformationen und den zweiten Elektroenzephalogrammminformationen, der Typen der Artefakte und
Treffen der Entscheidung über die Artefakte auf der Basis eines Verhältnisses einer Merkmalmenge der Elektroenzephalogrammminformationen während jeder von einer Mehrzahl erster Zeitspannen (T1) zu einer Merkmalmenge der Elektroenzephalogrammminformationen während einer zweiten Zeitspanne (T2), wobei die zweite Zeitspanne (T2) die Mehrzahl erster Zeitspannen (T1) umfasst, die so festgelegt sind, sich voneinander durch eine vorbestimmte Zeitmenge einer Zeitachse entlang zu verschieben,
wobei die Merkmalmenge ein integrierter Wert von Spektrumintensitäten der Elektroenzephalogrammminformationen auf einem vorbestimmten Frequenzband ist.

5. Computerproramm umfassend Anweisungen zum Verursachen des Elektroenzephalogrammmesssystems (10) nach Anspruch 1, um das Elektroenzephalogrammmessverfahren nach Anspruch 4 auszuführen.

6. Nichtvorübergehendes computerlesbares Speichermedium, das das Programm nach Anspruch 5 speichert.

## Revendications

1. Système de mesure d'électroencéphalogramme (10) comprenant :
une unité d'acquisition (211) configurée pour acquérir des informations d'électroencéphalogramme, les informations d'électroencéphalogramme représentant un électroencéphalogramme obtenu par une unité d'électrodes (11), l'unité d'électrodes (11) étant placée sur une région d'intérêt (51) faisant partie de la tête d'un sujet (52) ;
une unité de décision (214) configurée pour prendre une décision, sur la base des informations d'électroencéphalogramme acquises par l'unité d'acquisition (211), concernant la présence ou l'absence de quelconques artefacts ;
une unité de sortie (217) configurée pour fournir en sortie la décision prise par l'unité de décision (214) ;
une unité d'entrée (216) configurée pour accepter un signal de désignation désignant un ou plusieurs ensembles des informations d'électroencéphalogramme, parmi plusieurs ensembles des informations d'électroencéphalogramme acquises par l'unité d'acquisition (211), le signal de désignation étant entré par l'intermédiaire d'une unité d'exploitation (24) ; et
une unité de traitement (215) configurée pour effectuer, sur la base du ou des ensembles des informations d'électroencéphalogramme désignés par le signal de désignation, un traitement d'étalonnage pour déterminer un paramètre à utiliser pour analyser les informations d'électroencéphalogramme,
dans lequel le système de mesure d'électroencéphalogramme (10) est configuré de sorte qu'un écran de sélection (203) est affiché sur une unité d'affichage (26),
dans lequel l'écran de sélection (203) comprend une zone d'affichage d'enregistrement (G41), la zone d'affichage d'enregistrement (G41) étant une zone pour afficher les électroencéphalogrammes,
dans lequel le système de mesure d'électroencéphalogramme (10) est configuré de sorte que, sous chaque électroencéphalogramme affiché en tant qu'enregistrement dans la zone d'affichage d'enregistrement (G41), une marque d'artefact (M2) en forme de bande est affichée en association avec chaque enregistrement,
dans laquelle la marque d'artefact (M2) est montrée pendant une période au cours de laquelle il est déterminé qu'un artefact est inclus dans les informations d'électroencéphalogramme, de sorte que la décision prise par l'unité de décision (214) est reflétée sur la marque d'artefact (M2),
dans lequel
lorsque la décision prise par l'unité de décision (214) est qu'il y a un artefact, l'unité de décision (214) est configurée pour déterminer en outre si un type de l'artefact est un artefact dérivé d'un mouvement des yeux ou un artefact dérivé d'un mouvement du corps,
dans lequel
la région d'intérêt (51) comprend une première région d'intérêt (511) et une deuxième région d'intérêt (512),
l'unité d'électrodes (11) comprend une première électrode (111) correspondant à la première région d'intérêt (511) et une deuxième électrode (112) correspondant à la deuxième région d'intérêt (512),
l'unité d'acquisition (211) est configurée pour acquérir des premières informations d'électroencéphalogramme représentant un électroencéphalogramme obtenu par la première électrode (111) et des deuxièmes informations d'électroencéphalogramme représentant un électroencéphalogramme obtenu par la deuxième électrode (112), et
l'unité de décision (214) est configurée pour déterminer, sur la base des premières informations d'électroencéphalogramme ou des deuxièmes informations d'électroencéphalogramme, et d'une différence entre les premières informations d'électroencéphalogramme et les deuxièmes informations d'électroencéphalogramme, les types des artefacts,
dans lequel
l'unité de décision (214) est configurée pour prendre la décision concernant les artefacts sur la base d'un rapport entre une quantité de caractéristiques des informations d'électroencéphalogramme pendant chacune d'une pluralité de premières périodes (T1) et une quantité de caractéristiques des informations d'électroencéphalogramme pendant une deuxième période (T2), la deuxième période (T2) comprenant la pluralité de premières périodes (T1) qui sont définies de manière à se décaler les unes par rapport aux autres d'une quantité de temps prédéterminée le long d'un axe temporel,
dans lequel
la quantité de caractéristiques est une valeur intégrée d'intensités spectrales des informations d'électroencéphalogramme sur une bande de fréquence prédéterminée.

2. Système de mesure d'électroencéphalogramme (10) selon la revendication 1, comprenant en outre une unité de commande d'affichage (218) configurée pour amener une unité d'affichage (26) à afficher la décision fournie en sortie par l'unité de sortie (217).

3. Système de mesure d'électroencéphalogramme (10) selon la revendication 2, dans lequel
l'unité de commande d'affichage (218) est configurée pour amener l'unité d'affichage (26) à afficher, avec la décision, une forme d'onde d'électroencéphalogramme dérivée par un procédé de dérivation différent d'un procédé de dérivation pour dériver une forme d'onde d'électroencéphalogramme utilisé pour prendre la décision.

4. Procédé de mesure d'électroencéphalogramme, comprenant le fait de :
acquérir des informations d'électroencéphalogramme représentant un électroencéphalogramme obtenu par une unité d'électrodes (11), l'unité d'électrodes (11) étant placée sur une région d'intérêt (51) faisant partie de la tête d'un sujet (52) ;
prendre une décision, sur la base des informations d'électroencéphalogramme acquises, concernant la présence ou l'absence de quelconques artefacts ;
fournir en sortie la décision prise concernant les artefacts ;
accepter un signal de désignation désignant un ou plusieurs ensembles des informations d'électroencéphalogramme, parmi plusieurs ensembles des informations d'électroencéphalogramme, le signal de désignation étant entré par l'intermédiaire d'une unité d'exploitation (24) ;
effectuer, sur la base du ou des ensembles des informations d'électroencéphalogramme désignés par le signal de désignation, un traitement d'étalonnage pour déterminer un paramètre à utiliser pour analyser les informations d'électroencéphalogramme,
afficher un écran de sélection (203) sur une unité d'affichage (26),
dans lequel l'écran de sélection (203) comprend une zone d'affichage d'enregistrement (G41), la zone d'affichage d'enregistrement (G41) étant une zone pour afficher les électroencéphalogrammes,
dans lequel, sous chaque électroencéphalogramme affiché en tant qu'enregistrement dans la zone d'affichage d'enregistrement (G41), une marque d'artefact (M2) en forme de bande est affichée en association avec chaque enregistrement,
dans laquelle la marque d'artefact (M2) est montrée pendant une période au cours de laquelle il est déterminé qu'un artefact est inclus dans les informations d'électroencéphalogramme, de sorte que la décision prise par l'unité de décision (214) est reflétée sur la marque d'artefact (M2),
lorsque la décision est qu'il y a un artefact, déterminer en outre si un type de l'artefact est un artefact dérivé d'un mouvement des yeux ou un artefact dérivé d'un mouvement du corps,
dans lequel
la région d'intérêt (51) comprend une première région d'intérêt (511) et une deuxième région d'intérêt (512),
l'unité d'électrodes (11) comprend une première électrode (111) correspondant à la première région d'intérêt (511) et une deuxième électrode (112) correspondant à la deuxième région d'intérêt (512),
dans lequel le procédé comprend en outre le fait de
acquérir des premières informations d'électroencéphalogramme représentant un électroencéphalogramme obtenu par la première électrode (111) et des deuxièmes informations d'électroencéphalogramme représentant un électroencéphalogramme obtenu par la deuxième électrode (112), et
déterminer, sur la base des premières informations d'électroencéphalogramme ou des deuxièmes informations d'électroencéphalogramme, et d'une différence entre les premières informations d'électroencéphalogramme et les deuxièmes informations d'électroencéphalogramme, les types des artefacts, et
prendre la décision concernant les artefacts sur la base d'un rapport entre une quantité de caractéristiques des informations d'électroencéphalogramme pendant chacune d'une pluralité de premières périodes (T1) et une quantité de caractéristiques des informations d'électroencéphalogramme pendant une deuxième période (T2), la deuxième période (T2) comprenant la pluralité de premières périodes (T1) qui sont définies de manière à se décaler les unes par rapport aux autres d'une quantité de temps prédéterminée le long d'un axe temporel,
dans lequel la quantité de caractéristiques est une valeur intégrée d'intensités spectrales des informations d'électroencéphalogramme sur une bande de fréquence prédéterminée.

5. Programme informatique comprenant des instructions pour amener le système de mesure d'électroencéphalogramme selon la revendication 1 à mettre en œuvre le procédé de mesure d'électroencéphalogramme selon la revendication 4.

6. Support de stockage non transitoire lisible par ordinateur stockant le programme selon la revendication 5.
